(19) Europäisches Patentamt
European Patent Office
Office européen des brevets

(11) **EP 4 169 903 A1**

(12) **EUROPEAN PATENT APPLICATION**

(43) Date of publication:
**26.04.2023 Bulletin 2023/17**

(21) Application number: **21204515.7**

(22) Date of filing: **25.10.2021**

(51) International Patent Classification (IPC):
***C07C 229/26*** *(2006.01)* ***C07C 57/30*** *(2006.01)*
***C07C 229/28*** *(2006.01)* ***A61P 29/00*** *(2006.01)*

(52) Cooperative Patent Classification (CPC):
**A61P 29/00; C07C 57/30; C07C 57/58; C07C 59/64; C07C 59/68; C07C 229/26; C07C 229/28;** C07B 2200/13; C07C 2601/14

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Dompe' Farmaceutici S.P.A.**
**20122 Milan (IT)**

(72) Inventors:
- **ARAMINI, Andrea**
**67100 L'Aquila (IT)**
- **BIANCHINI, Gianluca**
**67100 L'Aquila (IT)**
- **LILLINI, Samuele**
**80131 Napoli (IT)**
- **TOMASSETTI, Mara**
**80131 Napoli (IT)**
- **BRANDOLINI, Laura**
**67100 L'Aquila (IT)**

(74) Representative: **Mauri, Elisabetta Maria Ester**
**Dompé Farmaceutici S.p.A.**
**Via Santa Lucia, 6**
**20122 Milano (IT)**

(54) **CO-CRYSTALS OF NONSTEROIDAL ANTI-INFLAMMATORY DRUGS, LYSINE AND GABAPENTIN, PHARMACEUTICAL COMPOSITIONS AND THEIR MEDICAL USE**

(57) The present invention relates to new co-crystals of non-steroidal anti-inflammatory drugs (NSAIDs) belonging to the class of phenylpropionic or phenylacetic acid derivatives, Lysine and Gabapentin, to their pharmaceutical compositions and to their use in the prevention, reduction or treatment of pain and/or inflammation.

FIG.1

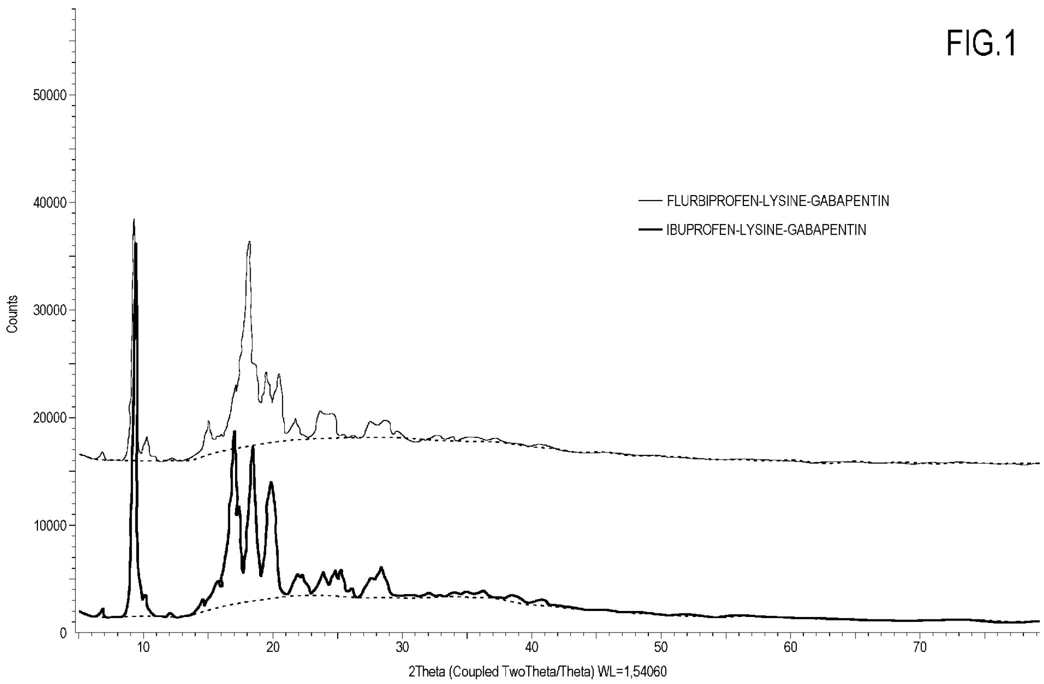


EP 4 169 903 A1

## Description

The present invention relates to co-crystals of Nonsteroidal anti-inflammatory drugs (NSAIDs) belonging to the class of phenylpropionic or phenylacetic acids, Lysine and Gabapentin, to a process for their preparation, to a pharmaceutical composition comprising said co-crystals and to the use of said co-crystals or pharmaceutical compositions in the treatment of acute or chronic pain, in particular in the treatment of neuropathic or inflammatory pain.

BACKGROUND ART

Pain is the mean by which the body signals tissue damage.

According to the definition of the IASP (International Association for the Study of Pain - 2020) and the World Health Organization, pain is an "unpleasant sensory and emotional experience associated with, or resembling that associated with, actual or potential tissue damage".

The experience of pain is different for every person, and there are various ways to feel and describe it.

Generally, pain conditions can be divided in acute and chronic.

Acute pain is an intense pain that lasts for a short period of time, typically less than 3 months, and is commonly associated with tissue injury or localized tissue damage and inflammation. In particular, there are three different types of acute pain: somatic pain (a person feels pain on the skin or the soft tissues just below the skin), visceral pain (pain in the internal organs and the linings of cavities in the body) and referred pain (pain referred at a location other than the source of tissue damage).

Chronic pain lasts far longer than acute pain, usually more than 3 months. Chronic pain lacks the acute warning function of physiological nociception. In general, chronic pain can be mild or severe, continuous (such as in arthritis) or intermittent (such as with migraines).

In particular, the peripheral nervous system (PNS) and central nervous system (CNS) are responsible for generating and sustaining the sensitization of nociceptive neurons leading to chronic pain.

Chronic pain may have different etiologies and includes neuropathic pain, chronic inflammatory pain, for example arthritis, or pain of unknown origin, as fibromyalgia and restless leg syndrome.

Chronic neuropathic pain is caused by a lesion or disease of the somatosensory nervous system that provides information about the body including skin, musculoskeletal, and visceral organs.

The pharmacological treatment of chronic inflammatory pain usually includes the use of non-steroidal anti-inflammatory drugs (NSAIDs).

Non-steroidal anti-inflammatory drugs are commonly grouped, according to the chemical structure, in different classes, for instance in salicylates such as Aspirin, Sodium Salicylate and Diflunisal, propionic acid derivatives such as Ibuprofen, Ketoprofen, Flurbiprofene and Naproxen, acetic acid derivatives such as Diclofenac, Ketorolac, Indomethacin, Sulindac and Zomepirac, alkanones such as Nabumetone and oxicams such as Piroxicam and Tenoxicam (J Clin Pharmacol 1988 Jun;28(6):512-7).

NSAIDs have antipyretic, analgesic, and anti-inflammatory effects, obtained thanks to the inhibition of prostaglandin synthesis. In particular, NSAIDs inhibit cyclooxygenases (COXs) which are the enzymes that catalyze the synthesis of cyclic endoperoxides from arachidonic acid to form prostaglandins. Non-selective NSAIDs inhibit the activity of both COX-1 and COX-2, whereas selective COX-2 inhibit only cyclooxygenase COX2.

Although NSAIDs are commonly used, they can cause troublesome side effects as increased risk of gastrointestinal ulcers and bleeds, heart attack, and kidney disease. There have been a number of attempts to eliminate these side effects. However, as most of NSAIDs suppress the production of prostaglandins involved in limiting the secretion of gastric acid, it has been difficult to completely eliminate this side effect. Therefore, today a new therapy for the treatment of chronic pain not causing the above mentioned severe side effects is required.

Chronic inflammatory pain conditions are often associated with neuroinflammation which is a physiological/pathological condition characterized by infiltration of immune cells, namely leukocytes releasing inflammatory cytokines. Neuroinflammation also implies activation of glial cells and production of inflammatory mediators which modulate the pain sensitivity. In particular, Schwann cells are activated in the nerve, satellite glial cells in the ganglia and microglia, and astrocytes and oligodendrocytes in the spinal cord and brain.

Today there is no effective therapy for the treatment neuroinflammatory conditions. Non-steroidal anti-inflammatory drugs are largely ineffective in neuropathic pain. Gabapentin is an anticonvulsant synthetic analogue of the neurotransmitter gamma-aminobutyric acid (GABA) of formula

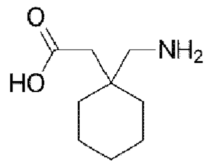

Gabapentin, is an anticonvulsant medication primarily used to treat partial seizures and neuropathic pain. Gabapentin has recently been approved for the treatment of chronic pain, in particular neuropathic pain conditions. Neuropathic pain is caused by a lesion or disease of the somatosensory system, including peripheral fibres and central neurons, and affects 7-10% of the general population. In particular, neuropathic pain is caused by imbalances between excitatory and inhibitory somatosensory signaling, alterations in ion channels and in the release of modulators.

Neuropathic pain is a chronic pain characterized by complex symptoms, poor outcomes and difficult treatment decisions.

The mechanism of action of Gabapentin includes binding to calcium channels in several areas of the central nervous system and spinal cord in which these channels are expressed. Calcium channels are localized on presynaptic terminals, where they control neurotransmitter release.

Gabapentin is neither inhibited nor metabolized by hepatic enzymes. It is expelled by the renal system and its excretion half-life is roughly 6 hours.

Thus, Gabapentin is characterized by short half-life and, as a consequence, need to be administered three times a day (tds).

Rapid titration can be achieved with doses of 300 mg once daily (often at bedtime to minimize sedation) on the first day followed by 300 mg twice daily on the second day and 300 mg tds on the third day. Dosage can be further increased if efficacy is not achieved at this dose.

An unsatisfactory pharmacological and pharmacokinetic profile has been observed when Gabapentin is used alone in pain therapy. In other words, when Gabapentin is used in monotherapy for the treatment of pain is not completely effective. Indeed, a delayed onset of the response was registered. Gabapentin is absorbed slowly after oral administration, and it has an utmost level in plasma within 3-4 hours (Quintero, Journal of Experimental Pharmacology 2017:9 13-21). Furthermore, Gabapentin is little active on inflammatory pain, as also confirmed in the present experimental part in the Carrageenan inflammatory rat model.

In addition, Gabapentin is not recommended for the treatment of lower back pain (*Low back pain and sciatica in over 16s: assessment and management,* National Institute for Health and Care Excellence NICE Guidelines 2016).

Considering the disadvantages of using Gabapentin in monotherapy for the treatment of pain, the Applicant has undertaken several studies to improve the properties of Gabapentin for use in the treatment of pain conditions.

In particular, the Applicant has carried out investigations on Gabapentin combined with non-steroidal anti-inflammatory drugs, specifically with non-steroidal anti-inflammatory drugs belonging to the class of phenylpropionic and phenylacetic acids.

SUMMARY OF THE INVENTION

The Applicant with the aim to improve the therapy of pain conditions has surprisingly found that non-steroidal anti-inflammatory drugs belonging to the class of phenylpropionic or phenylacetic acids form stable co-crystals with Gabapentin and Lysine.

During these investigations, the Applicant has found that the co-crystals of non-steroidal anti-inflammatory drugs belonging to the above mentioned classes, Lysine and Gabapentin, show a surprising synergistic effect on inflammation and pain.

In fact, when these active ingredients are associated in the co-crystals of the invention, they show an anti-inflammatory and analgesic activity greater than that obtained with the co-administration of the separated actives (NSAID, Gabapentin) Hence the present co-crystals allow to use lower therapeutic doses of either NSAID or Gabapentin or both, thus minimizing the side effects. Additionally, in comparison to Gabapentin alone, a prolongation of the efficacy over time was observed. Finally, the co-crystals improves dissolution rates of NSAIDs, especially if dissolving in an aqueous physiological surrounding, and enhances the absorption and/or the bioavailability of the two active molecules Gabapentin and NSAID.

It is thus an object of the present invention a co-crystal of a nonsteroidal anti-inflammatory drug (NSAID) belonging to the class of phenylpropionic or phenylacetic acids, Lysine and Gabapentin, providing that said nonsteroidal anti-inflammatory drug is not Ketoprofen.

According to an embodiment of the invention, said non-steroidal anti-inflammatory drug is a phenylpropionic acid derivative selected from Ibuprofen, Flurbiprofen, Fenoprofen, Indoprofen, Loxoprofen, Pelubiprofen, and Naproxen.

According to an embodiment of the invention, said non-steroidal anti-inflammatory drug is a phenylacetic acid derivative selected from Diclofenac, Felbinac, Ibufenac, Fenclofenac, Tifurac, and Ketorolac.

According to a particularly preferred embodiment of the invention, said non-steroidal anti-inflammatory drug is selected from Ibuprofen and Flurbiprofen.

The co-crystals according to the invention of Flurbiprofen-Lysine-Gabapentin (FLG) and Ibuprofen-Lysine-Gabapentin (ILG) are respectively characterized by the XRPD diffractograms reported in Figure 1 and by XRPD positions and intensities depicted in Tables 2-3.

The Applicant has surprisingly observed that the crystals of Flurbiprofen-Lysine-Gabapentin and of Ibuprofen-Lysine-Gabapentin were of striking resemblance to each other, with the reflexes of the co-crystals having similar intensities of the respective main peaks in the region between 9 - 10 degrees 2-theta, 15 - 25 degrees 2-theta and 27 - 28 degree 2-theta. The same pattern was observed with a previous co-crystal of Ketoprofen - Lysine - Gabapentin, described in international patent application PCT/EP2021/060421, which is not part of the present invention.

This similarity was taken as clear evidence of isostructurality of the co-crystals. Isostructurality refers to different components with similar crystal packing. When closely related molecules are introduced in the crystal lattice of a single component, the corresponding binary system may exhibit close packing [G. Portalone, Crystals, 2020, 10, 999-1012].

A further object of the present invention is a process for the preparation of the co-crystals of the invention, which comprises:

a) suspending a non-steroidal anti-inflammatory drug (NSAID) belonging to the class of phenylpropionic or phenylacetic acids, Lysine, and Gabapentin in a suitable solvent,
b) dissolving said NSAID, Lysine, and Gabapentin, optionally by heating the suspension, possibly under stirring, until a clear solution is obtained,
c) optionally cooling the solution obtained, and/or
d) optionally adding an anti-solvent, thus providing the NSAID, Lysine, and Gabapentin co-crystal.

A further object of the present invention is a pharmaceutical composition comprising the co-crystals of the invention and at least a pharmaceutically acceptable excipient. A further object of the present invention is a pharmaceutical composition comprising a co-crystal according to the invention and at least another pharmaceutically active ingredient.

A further object of the present invention is a co-crystals according to the invention for use in the treatment of pain and/or inflammation.

A further object of the present invention is a method for the treatment of pain and/or inflammation comprising administering to the patient an effective amount of a co-crystal according to of the invention.

DEFINITIONS

For the purpose of the present invention, the term "co-crystal" means a multicomponent system, in which all components are solid under ambient conditions when in their pure form. The components coexist at a molecular level within a single crystal. At least some the components are connected by non-covalent, non-ionic interactions.

For the purpose of the present invention, the term "pain" means pain caused by disturbances of different nature and origin, such as, for example: headache or cephalalgia: both primary and therefore not related to other factors or diseases, and secondary and therefore dependent on trauma, injury and distinct diseases; toothache: in case of abscesses or caries that create pain in the dental pulp, with numerous blood vessels and nerves; menstrual pains: abdominal and lower abdominal pain and headaches caused by hormonal changes typical of the period of menstruation; neuralgia, or intense nerve pain due to strains, trauma and infections; pain in the muscles, or myalgia: pains located at the level of muscles when using or touching them, due to sudden contractions or traumas; osteoarticular pains, such as joint inflammations (to the bones, cartilages, ligaments and tendons) following traumas, old age, strains and injuries.

For the purpose of the present invention, the term "inflammation" means the local response of an organism to cellular injury that is marked by capillary dilatation, leukocytic infiltration, redness, heat, and pain and that serves as a mechanism initiating the elimination of noxious agents and of damaged tissue.

For the purpose of the present invention, the term "pharmaceutically acceptable excipient" refers to a substance devoid of any pharmacological effect of its own and which does not produce adverse reactions when administered to a mammal, preferably a human.

For the purpose of the present invention, the term "room temperature" means a temperature range of 18 to 25°C.

For the purpose of the present invention, the term "anti-solvent" means a solvent in which a compound is insoluble or little soluble.

In the present description and in the figures attached to the description, the abbreviation "Gaba" indicates Gabapentin.

The terms "approximately" and "about" herein refers to the range of the experimental error, which may occur

in a measurement.

BRIEF DESCRIPTION OF THE FIGURES

Figure 1: Powder X-Ray diffraction pattern of Flurbiprofen-Lysine-Gabapentin and Ibuprofen-Lysine-Gabapentin co-crystals.
Figure 2: DSC thermogram of Flurbiprofen-Lysine-Gabapentin 1:1:1 co-crystal.
Figure 3: DSC thermogram of Ibuprofen-Lysine-Gabapentin 1:1:1 co-crystal.
Figure 4: TG thermograms of Flurbiprofen-Lysine-Gabapentin 1:1:1 co-crystal.
Figure 5: TG thermograms of Ibuprofen-Lysine-Gabapentin 1:1:1 co-crystal.
Figure 6: Raman spectrum of Flurbiprofen-Lysine-Gabapentin 1:1:1 co-crystal.
Figure 7: Raman spectrum of Ibuprofen-Lysine-Gabapentin 1:1:1 co-crystal.
Figure 8: FT-IR spectrum of Flurbiprofen-Lysine-Gabapentin 1:1:1 co-crystal.
Figure 9: FT-IR spectrum of Ibuprofen-Lysine-Gabapentin 1:1:1 co-crystal.
Figure 10: $^{1}$H-NMR spectrum (400 MHz, $D_2O$) of Flurbiprofen-Lysine-Gabapentin 1:1:1 co-crystal.
Figure 11: $^{1}$H-NMR spectrum (400 MHz, $D_2O$) of Ibuprofen-Lysine-Gabapentin 1:1:1 co-crystal.
Figure 12: $^{13}$C CPMAS spectra of Flurbiprofen-Lysine-Gabapentin 1:1:1 co-crystal.
Figure 13: Detail of the carboxylic region of the 13C CPMAS spectra of FLU-LYS-GAB, FLU·SALA, FLU·PICA, NaFLU, pure FLU, pure GAB, NaGAB, DL-LYS·2HCl, L-LYS acetate and pure L-LYS.
Figure 14. 15N CPMAS spectra of Flurbiprofen-Lysine-Gabapentin 1:1:1 co-crystal.
Figure 15. Comparison among the 15N CPMAS spectra of FLU-LYS-GAB, NaGAB, GAB, DL-LYS·2HCl, L-LYS acetate and pure L-LYS.
Figure 16: $^{13}$C CPMAS spectra of Ibuprofen-Lysine-Gabapentin 1:1:1 co-crystal.
Figure 17: Detail of the carboxylic region of the $^{13}$C CPMAS spectra of IBU-LYS-GAB, IBU·L-proline, NaIBU, pure IBU, pure GAB, NaGAB, DL-LYS·2HCl, L-LYS acetate and pure L-LYS.
Figure 18. 15N CPMAS spectra of Ibuprofen-Lysine-Gabapentin 1:1:1 co-crystal.
Figure 19. Comparison among the 15N CPMAS spectra of IBU-LYS-GAB, NaGAB, GAB, DL-LYS·2HCl, L-LYS acetate and pure L-LYS.
Figure 20. 13C (150.91 MHz) CPMAS spectra of Flurbiprofen-Lysine-Gabapentin and Ibuprofen-Lysine-Gabapentin, acquired at room temperature at a spinning speed of 20 kHz.
Figure 21. 15N (60.83 MHz) CPMAS spectra of Flurbiprofen-Lysine-Gabapentin and Ibuprofen-Lysine-Gabapentin, acquired at room temperature at a spinning speed of 12 kHz.
Figure 22: Effect of Flur/Lys/Gaba co-crystal in comparison with the admixture of Flur + Lys + Gaba admixture and with single actives, respectively Flurbiprofen, Gabapentin and Indomethacin, on carrageenan-induced rat paw edema. In the present Figure "Gaba" indicates Gabapentin.
Figure 23: Effect of Flur/Lys/Gaba co-crystal in comparison with the admixture of Flur + Lys + Gaba admixture and with single actives, respectively Flurbiprofen, Gabapentin and Indomethacin, on mechanical allodynia in a rat carrageenan-induced paw edema model. In the present Figure "Gaba" indicates Gabapentin.

DETAILED DESCRIPTION OF THE INVENTION

An object of the present invention is a co-crystal of a nonsteroidal anti-inflammatory drug (NSAID) belonging to the class of phenylpropionic or phenylacetic acids, Lysine and Gabapentin, providing that said nonsteroidal anti-inflammatory drug is not Ketoprofen.

According to a preferred embodiment of the invention, the molar ratio of the components in the co-crystal is 1:1:1.

In line with the analysis reported in the experimental part, in the co-crystal according to the invention the carboxylic group of the non-steroidal anti-inflammatory drug (NSAID) belonging to the class of phenylpropionic or phenylacetic acids is deprotonated and interacts with protonated Lysine $\varepsilon$-$NH_3^+$ group through ionic bonds forming a neutral salt.

The salt of Lysine with the non-steroidal anti-inflammatory drug (NSAID) belonging to the class of phenylpropionic or phenylacetic acids, in particular Flurbiprofen or Ibuprofen salt with Lysine, interacts with Gabapentin through non-ionic bonds, forming stable co-crystals.

According to an embodiment of the invention, said non-steroidal anti-inflammatory drug is a phenylpropionic acid derivative selected from Ibuprofen, Flurbiprofen, Fenoprofen, Indoprofen, Loxoprofen, Pelubiprofen, and Naproxen.

According to an embodiment of the invention, said non-steroidal anti-inflammatory drug is a phenylacetic acid derivative selected from Diclofenac, Felbinac, Ibufenac, Fenclofenac, Tifurac, and Ketorolac.

In the co-crystal according to the invention, the chiral carbon of the NSAID, when present, can be optically pure (S) or (R) or racemic (S,R) or in form of any admixture of the stereoisomers.

In the co-crystal of the invention, Lysine can be racemic (S,R) Lysine, (S) Lysine or (R) Lysine, or any admixture thereof, preferably is the natural aminoacid (S)-Lysine also named L-Lysine.

According to a particularly preferred embodiment, said nonsteroidal anti-inflammatory drug is selected from Flurbiprofen and Ibuprofen. According to another preferred embodiment, said nonsteroidal anti-inflammatory drug is Flurbiprofen.

In the co-crystal according to this embodiment, Flurbiprofen can be racemic (S,R) Flurbiprofen, (S)-Flurbiprofen or (R)- Flurbiprofen or any admixture thereof.

In the co-crystal according to this embodiment,, Lysine can be racemic (S,R) Lysine, (S) Lysine or (R) Lysine, or any admixture thereof, preferably is the natural aminoacid (S)-Lysine also named L-Lysine.

In one embodiment, the co-crystal of the invention comprises (S,R)- Flurbiprofen. In one embodiment, the co-crystal of the invention comprises (S,R)-Lysine.

In one embodiment, the co-crystal of the invention comprises (S,R)- Flurbiprofen and (S,R)-Lysine.

According to another preferred embodiment, said nonsteroidal anti-inflammatory drug is Ibuprofen.

In the co-crystal according to this embodiment, Ibuprofen can be racemic (S,R) Ibuprofen, (S)- Ibuprofen or (R)- Ibuprofen or any admixture thereof.

Also, in the co-crystal according to this embodiment, Lysine can be racemic (S,R) Lysine, (S) Lysine or (R) Lysine, or any admixture thereof, preferably is the natural aminoacid (S)-Lysine also named L-Lysine.

In one embodiment, the co-crystal of the invention comprises (S,R)- Ibuprofen.

In one embodiment, the co-crystal of the invention comprises (S,R)- Lysine.

In one embodiment, the co-crystal of the invention comprises (S,R)- Ibuprofen and (S,R)-Lysine.

The co-crystal of the present invention can exist in non-solvated forms as well as solvated forms, including hydrated forms.

The co-crystals of the present invention are easily obtainable and stable.

The co-crystals of the present invention show improved pharmaceutical properties, pharmacokinetics and efficacy in pain conditions, as described in the Experimental section that follows.

The co-crystals of the present invention are characterized by a common XRPD pattern, in particular by the following common XRPD diffraction peaks: 9.3, 17.1, 18.5, 19.8, 22.1, 24.1, 24.9, 27.9 degrees 2-theta ± 0.2 degrees 2-theta.

Other polymorphs of the present co-crystals are also within the scope of the invention.

According to an embodiment, the co-crystal of Flurbiprofen, Lysine and Gabapentin of the present invention is characterized by the following XRPD diffraction peaks: 9.3, 10.4, 15.2, 16.0, 17.2, 18.3, 18.8, 19.7, 20.7, 21.9, 24.0, 24.8, 27.9, and 29.0 degrees 2-theta ± 0.2 degrees 2-theta, preferably further characterized by the following XRPD diffraction peaks: 6.9, 10.9, 12.2, 25.5, 26.3, 29.8, 31.5, 33.0, 34.0, 35.9, 37.5, 39.2 and 40.8 degrees 2-theta ± 0.2 degrees 2-theta.

According to this embodiment, the co-crystal of co-crystal of Flurbiprofen, Lysine and Gabapentin of the present invention is further characterized by the DSC thermogram of Figure 2, with the endothermic sharp peak of the co-crystal corresponding to the melting point at 163.48°C with an onset at 161.59°C, the TGA thermogram of Figure 4, FT Raman and FT-IR spectra with typical absorption bands reported in Figures 6 and 8, solution $^1$H-NMR spectrum of Figure 10 and relative assignments in Table 10, solid state $^{13}$C CPMAS of Figure 12, 13 and relative assignments in Table 12 and/or 15N CPMAS spectra of Figures 14 and 15. According to another embodiment, the co-crystal of Ibuprofen, Lysine and Gabapentin of the present invention is characterized by the following XRPD diffraction peaks: 9.5, 10.3, 15.9, 17.1, 17.6, 18.7, 20.0, 22.3, 24.1, 25.1, 25.6, 27,9 and 28.6 degrees 2-theta ± 0.2 degrees 2-theta, preferably further characterized by the following XRPD diffraction peaks: 6.9, 12.0, 14.7, 26.3, 30.6, 31.1, 32.3, 33.1, 34.5, 35.3, 36.6, 38.6, 39.0, 41.1, and 48.9 degrees 2-theta ± 0.2 degrees 2-theta. According to this embodiment, the co-crystal Ibuprofen, Lysine and Gabapentin of the present invention is further characterized by the DSC thermogram of Figure 3, with the endothermic sharp peak of the co-crystal corresponding to the melting point at 165.60°C with an onset at 161.60°C, the TGA thermogram of Figure 5, FT Raman and FT-IR spectra with typical absorption bands reported in Figures 7 and 9, solution $^1$H-NMR spectrum of Figure 11 and relative assignments in Table 11, solid state $^{13}$C CPMAS of Figures 16, 17 and relative assignments in Table 13 and/or 15N CPMAS spectra of Figures 18 and 19.

It has been observed that the co-crystals having the XRPDs above indicated, in particular the co-crystals of Fluibrufen-Lysine-Gabapentin and of Ibuprofen-Lysine-Gabapentin, are isostructural co-crystals.

Isostructurality indicates different components with similar crystal packing. When closely related molecules are introduced in the crystal lattice of a single component, the corresponding binary system may exhibit close packing (G. Portalone, Crystals, 2020, 10, 999-1012).

Isostructural co-crystals with similar structural blueprints may exhibit common modified pharmaceutically relevant properties due to the presence of variable coformers and minor changes in their conformations and packing features.

Often, isostructural co-crystals have similar lattice parameters including space groups, defined as isomorphs, which generally exhibit identical X-ray diffraction pattern [F. Y. Wang, Q. Zhang, Z. Zhang, X. Gong, J. R. Wang and X. Mei, CrystEngComm, 2018, 20, 5945-5948]. The isostructurality of the co-crystals was demonstrated by XRPD pattern comparison and further confirmed by 13C and 15N-CPMAS NMR spectra (see Experimental section).

XRPD diffractograms of the present co-crystals are very similar, with the reflexes of the respective main peaks in the region between 14 and 22 degrees 2-theta having similar intensities (Figure 1 - XRPD).

This similarity was taken as clear evidence of isostructurality of the co-crystals. Furthermore, in the 13C and 15N-CPMAS NMR spectra of two co-crystals (see Figures 20 and 21) many of the resonances match coincides. Careful analysis of the signals points out that all the recurring signals in the aliphatic region (below 80 ppm) correspond to the aliphatic C nuclei of LYS and GAB. The most apparent differences are in the aromatic range (110-170 ppm), which is reasonable, since the aromatic carbons are those of the different NSAIDs. Most notably, the same similarities can be observed in the carboxylic region, with the carboxylic resonances of FLU-LYS-GAB and IBU-LYS-GAB.

The same remarkable spectral similarities can be observed in the 15N CPMAS spectra of the samples.

All these spectral analogies indicate that the crystalline systems of FLU-LYS-GAB and IBU-LYS-GAB are in a homogeneous phase and they are characterized by a very similar local environment, especially regarding LYS and GAB, and comparable vibrational behaviors.

A further object of the present invention is the co-crystal of the invention for use as a medicament. The medical use can be curative, prophylactic or palliative.

The Applicant observed that the association of the two active ingredients in the same crystals exhibits several advantages for the present medical use. First, the co-crystals comprising a NSAID, Lysine and Gabapentin behave as a single chemical entity, thus facilitating the treatments, and causing less side effects with respect to the treatment with Gabapentin and NSAID alone.

The co-crystals comprising a NSAID, Lysine and Gabapentin can be advantageously used in the prevention, reduction or treatment of pain and/or inflammation, said pain being acute or chronic pain.

Preferably said According to a preferred use, the co-crystals comprising a NSAID, Lysine and Gabapentin are used for neuropathic or inflammatory pain. Indeed, the association of two active ingredients into one unique species may allow for a better Pharmacokinetic/Pharmacodynamic (PKPD) including also a better penetration of the blood-brain barrier, which helps in the treatment of neuropathic pain. Preferably, said pain is selected from headache, toothache, menstrual pain, muscle pain, neuropathic pain, pain associated to neuroinflammation, diabetic neuropathy, cancer pain, osteoarthritis, low back pain, sciatalgia, fibromyalgia, trigeminal neuralgia, post-surgical and post-operative pain, post herpetic neuralgia, rheumatoid arthritis, ankylosing spondylitis, frozen shoulder, phantom limb pain and HIV pain.

According to an embodiment, the daily dosage of the co-crystal for humans preferably provides for Flurbiprofen in an amount between 50 and 250 mg per day, more preferably 100 and 200 mg per day, preferably from 1 to 4 times a day. According to another embodiment, the daily dosage of the co-crystals for humans preferably provides for Ibuprofen in an amount between 200 and 800 mg per day, more preferably 400 and 600 mg per day, preferably from 1 to 4 times a day.

The daily dosage of the co-crystal according to the invention for humans advantageously results in a total amount of Gabapentin much lower if compared to the normal dosage of Gabapentin when this is used alone.

A further object of the present invention is a process for the preparation of the co-crystals of the invention, which comprises:

a) suspending a non-steroidal anti-inflammatory drug (NSAID) belonging to the class of phenylpropionic or phenylacetic acids, Lysine, and Gabapentin in a suitable solvent and stirring the resulting mixture at room temperature until a clear solution is obtained,

b) dissolving said NSAID, Lysine, and Gabapentin optionally by heating the suspension, possibly under stirring, until a clear solution is obtained,

c) optionally, cooling the solution obtained in b), and/or

d) optionally, adding an anti-solvent, thus providing the NSAID, Lysine, and Gabapentin co-crystal.

According to an embodiment of the invention, the non-steroidal anti-inflammatory drug according to step a) is a phenylpropionic acid derivative selected from Ibuprofen, Flurbiprofen, Fenoprofen, Indoprofen, Loxoprofen, Pelubiprofen, and Naproxen.

According to another embodiment of the invention, the non-steroidal anti-inflammatory drug is a phenylacetic acid derivative selected from Diclofenac, Felbinac, Ibufenac, Fenclofenac, Tifurac, and Ketorolac.

According to a preferred embodiment, said nonsteroidal anti-inflammatory drug is selected from Flurbiprofen, Ibuprofen, Diclofenac, and Naproxen, even more preferably from Ibuprofen and Flurbiprofen.

In the present process, the starting material for the NSAID can be a NSAID free acid or NSAID salt.

In case of NSAID free acid or a NSAID salt different from the Lysinate, Lysine is added, preferably in its neutral form. Lysine is preferably used in the same molar amount of the NSAID.

The NSAID salt may preferably be Flurbiprofen Lysinate or Ibuprofen Lysinate.

In step a) of the present process, the molar ratio of Gabapentin vs NSAID is preferably between 1:1 and 1.5:1, more preferably between 1:1 and 1.2:1, even more preferably is about 1:1.

In the preferred embodiment, the molar ratio of NSAID: Lysine: Gabapentin in step a) is about 1:1:1.

Suitable solvents to be used in step a) of the process according to the invention are water, alcohols, preferably methanol and ethanol, esters, preferably ethyl acetate, ethers, preferably tetrahydrofuran and tert-butylmethyl ether or aromatic solvents, preferably toluene.

Step b) can be carried out at room temperature or under heating, preferably at the temperature of reflux of the solvent.

Preferably the solution from step b) is kept or cooled at room temperature and filtered.

Preferably step b) is carried out under stirring. The stirring of step b) is performed preferably for 10 minutes to 30 minutes.

Optionally in step d) the precipitation of the co-crystal is favored by addition of an anti-solvent, such as ethyl acetate and tetrahydrofuran.

Preferably the anti-solvent can be added in a volumetric ratio from 1:1 to 16:1 with respect to the volume of the solution.

According to one embodiment, in step a) of the process according to the invention, NSAID and Lysine can be present as a pre-formed salt or co-crystal, in any polymorphic form.

The starting material for the manufacture of the co-crystals of the present invention can be prepared in accordance with methods of synthesis previously published and well known to the organic chemist of ordinary skill.

According to an alternative embodiment, said NSAID is a free acid and/or said Lysine is in neutral form.

In the present preparation process, Gabapentin is preferably used in its neutral form (zwitterionic internal salt) or in any acid or basic salified form, for instance as Gabapentin hydrochloride or Gabapentin Sodium salt.

Preferably, Gabapentin is used in its neutral form.

Gabapentin can be in any polymorph form.

The present process provides the co-crystals of the invention with high yields. It is simple and easy scalable at industrial level.

The present invention furthermore relates to a pharmaceutical composition comprising a co-crystal of a Non-steroidal anti-inflammatory drug (NSAID) belonging to the class of phenylpropionic or phenylacetic acids Lysine and Gabapentin as above described and a least one pharmaceutically acceptable excipient. According to an embodiment of the invention, said non-steroidal anti-inflammatory drug in the pharmaceutical composition is a phenylpropionic acid derivative selected from Ibuprofen, Flurbiprofen, Fenoprofen, Indoprofen, Loxoprofen, Pelubiprofen and Naproxen.

According to another embodiment of the invention, said non-steroidal anti-inflammatory drug in the pharmaceutical composition is a phenylacetic acid derivative selected from Diclofenac, Felbinac, Ibufenac, Fenclofenac, Tifurac and Ketorolac.

According to a particularly preferred embodiment, the pharmaceutical composition comprises co-crystals of Flurbiprofen-Lysine-Gabapentin.

According to another particularly preferred embodiment, the pharmaceutical composition comprises co-crystals of Ibuprofen-Lysine-Gabapentin.

The non-steroidal anti-inflammatory drug in the co-crystals present in the pharmaceutical composition of the invention is not Ketoprofen.

The composition according to the present invention may contain 6-60% by weight of the co-crystals as defined herein and 40-94 % by weight of one or more pharmaceutically acceptable excipients.

The choice of the excipients will to a large extent depend on factors such as the particular mode of administration, the effect on solubility and stability, and the nature of the dosage form.

Pharmaceutical compositions according to the present invention can be in any form suitable for the application to humans and/or animals, preferably humans including infants, children and adults and can be produced by standard procedures known to those skilled in the art.

The pharmaceutical composition of the present invention preferably is an oral solid composition, such as for instance a capsule, pellet, tablet, cachet, chewable dosage forms, powder, lozenge, granules, oral soluble granulate, suspension, emulsion, spray, or as dry powdered form to be reconstituted with a liquid medium.

The pharmaceutical composition can additionally contain one or more pharmaceutically acceptable excipients, such as fillers, binders, glidants, disintegrants, flow regulating agents and release agents.

Suitable excipients are for example disclosed in "Handbook of Pharmaceutical Excipients", 3rd Edition, published by A.H. Kibbe, American Pharmaceutical Association, Washington, USA, and Pharmaceutical Press, London.

Suitable fillers are for example lactose (monohydrate, spray-dried monohydrate, anhydrous and the like), mannitol, xylitol, dextrose, sucrose, sorbitol, microcrystalline cellulose, starch, dibasic calcium phosphate dihydrate and calcium hydrogen phosphate.

Fillers can be present in an amount of 0 - 80% by weight, preferably in an amount of 10 - 60% by weight of the total weight of the composition.

Suitable binders are for example polyvinylpyrrolidone, microcrystalline cellulose hydroxypropyl cellulose, hydroxypropyl methylcellulose, methylcellulose, hydroxyethyl cellulose, sugars, dextran, corn starch, gelatin, polyethylene glycol, natural and synthetic gums, pregelatinised starch.

Binders can be present in an amount of 0 - 80% by weight, preferably in an amount of 10 - 60% by weight of the total weight of the composition.

Binders are generally used to impart cohesive qualities to a tablet formulation. Suitable glidants are for example alkaline earth metal salts of fatty acids, like stearic acid such as magnesium stearate, calcium stearate, zinc stearate, sodium stearyl fumarate, and mixtures of magnesium stearate with sodium lauryl sulphate.

The glidant can be present for example in an amount of 0 - 2% by weight, preferably in an amount of 0.5 - 1.5% by weight of the total weight of the composition. Suitable disintegrants are for example crosscarmellose sodium, sodium carboxymethyl starch, crosslinked polyvinylpyrrolidone (crosspovidone), sodium carboxymethylglycolate, sodium starch glycolate, sodium carboxymethyl cellulose, calcium carboxymethyl cellulose, polyvinylpyrrolidone, methyl cellulose, microcrystalline cellulose, lower alkyl-substituted hydroxypropyl cellulose, starch, pregelatinised starch, sodium alginate and sodium bicarbonate.

The disintegrant can be present in an amount of 0 - 20% by weight, preferably in an amount of 1 - 15% by weight of the total weight of the composition.

A suitable flow regulating agent is for example colloidal silica. The flow regulating agent can be present in an amount of 0 - 8% by weight, preferably in an amount of 0.1 - 3% by weight of the total weight of this composition.

A suitable release agent is for example talcum. The release agent can be present in an amount of 0 - 5% by weight, preferably in an amount of 0.5 - 3% by weight of the total weight of the composition.

The solid composition can be coated, preferably film coated.

A suitable coating agent are for example cellulose derivatives, poly(meth)acrylate, polyvinyl pyrrolidone, polyvinyl acetate phthalate, and/or shellac or natural rubbers such as carrageenan.

The pharmaceutical composition of the invention can be a solid implant composition wherein the co-crystals are in form of a solid. This composition can be installed into body tissues or cavities.

The implant may comprise a matrix of bio-compatible and bioerodible materials in which particles of the co-crystals of the present invention are dispersed, or in which, possibly, globules or isolated cells of a liquid mixture of the present co-crystals are entrapped. Desirably, the matrix will be broken down and completely absorbed by the body. The composition of the matrix is also preferably selected to provide controlled-, sustained-, and/or delayed release of the co-crystals of the present invention over extended periods.

Alternatively, the co-crystals of the invention can be formulated as a solid, semisolid, or thixotropic liquid for administration as an implanted depot providing modified release of the active compound.

The present composition can be administered topically to the skin or mucosa, that is dermally, epidermally, subepidermally or transdermally.

The present composition can be administered sublingually or via a suppository.

Typical formulations for this purpose include pour-on, spot-on, dip, spray, mousse, shampoo, powder formulation, gels, hydrogels, lotions, creams, ointments, dusting powders, dressings, foams, films, skin patches, wafers, implants, depots, sponges, fibres, bandages, microemulsions, orosoluble granulates. Liposomes may also be used.

The pharmaceutical composition of the present invention can be a solid composition for the extemporaneous preparation of a solution for oral or parenteral administration, for example to be administered by intramuscular, intraperitoneal, or intravenous injection.

The pharmaceutical composition of the present invention can be prepared by methods well known to a person skilled in the art.

The composition of the invention can be of immediate-, delayed-, modified-, sustained-, pulsed- or controlled-release type.

According to a further embodiment, the pharmaceutical composition of the invention may comprise the co-crystals of the invention and at least another pharmaceutically active ingredient.

The other pharmaceutically active ingredient will be determined by the circumstances under which the therapeutic agent of the present invention is administered.

According to the invention, the pharmaceutical composition can be used for the prevention, reduction or treatment of pain and/or inflammation, said pain being acute or chronic pain.

According to a preferred use, the composition is used for the prevention, reduction and/or treatment of neuropathic or inflammatory pain.

Said pain is selected from headache, toothache, menstrual pain, muscle pain, neuropathic pain, pain associated to neuroinflammation, diabetic neuropathy, cancer pain, osteoarthritis, low back pain, sciatalgia, fibromyalgia, trigeminal neuralgia; post-surgical and post-operative pain, post herpetic neuralgia, rheumatoid arthritis, ankylosing spondylitis,

frozen shoulder, phantom limb pain or HIV pain.

A further object of the present invention is a method for the prevention, reduction or treatment of pain and/or inflammation comprising administering to a subject, preferably a human, in need thereof an effective amount of the co-crystals of the invention and/or of a pharmaceutical composition comprising said co-crystals.

In particular, said method allows the prevention, reduction or treatment of headache, toothache, menstrual pain, muscle pain, neuropathic pain, pain associated to neuroinflammation, diabetic neuropathy, cancer pain, osteoarthritis, low back pain, sciatalgia, fibromyalgia, trigeminal neuralgia; post-surgical and post-operative pain, post herpetic neuralgia, rheumatoid arthritis, ankylosing spondylitis, frozen shoulder, phantom limb pain or HIV pain. In particular, the patient would benefit from a longer duration of action from the co-crystals treatment than from treatment with Gabapentin or with NSAID or with their combination.

It is within the normal skills of the skilled artisan, such as a physician, to determine the preferred route of administration, the corresponding suitable dosage form and dose regimen.

For example, the daily dosage for humans and animals may vary depending on factors such as age, sex, weight or degree of illness and so forth.

According to an embodiment of the method for the prevention, reduction or treatment of pain and/or inflammation, the co-crystals and/or the pharmaceutical composition can be administered in combination with one or more other drugs useful for the prevention, reduction or treatment of pain and/or inflammation.

## EXPERIMENTAL PART

In the following, the manufacture of the co-crystals of NSAIDs, Lysine and Gabapentin, their analytical and biological characterization are described.

### 1. Synthesis of the co-crystal NSAID-Lysine-Gabapentin

The NSAID (3.3 mmol, 1.0 eq) was dissolved in 6 mL of ethanol. To the resulting solution, 50% w/w D,L-Lysine aqueous solution (1.0 eq.) was added and the solution stirred for 10 minutes. Then, 1.0 eq of Gabapentin was added. The solid precipitation took place in approximatively 30 minutes and the suspension was stirred at 25 °C for 5 hours (300 rpm). The solid product was isolated by vacuum filtration on a paper filter and then squeezed under a nitrogen flow for approximatively 10 minutes. The solid was gently ground and then dried at 40°C and 30 mbar overnight affording the desired product as a white solid of NSAID-Lysine-Gabapentin co-crystal (Yield: 75 - 90%).

The co-crystals of Flurbiprofen-Lysine-Gabapentin and of Ibuprofen-Lysine-Gabapentin were synthesized according to the above procedure and subjected to the following analyses.

### 2. XRPD Analysis

The XRPD analysis was carried out with the following instrument and under the conditions reported in Table 1 below:

Table 1

| | |
|---|---|
| Instrument type: | Rigaku MiniFlex600 |
| Application SW: | Miniflex Guidance |
| Measurement Details | |
| Measurement type: | Single scan |
| Sample mode: | Reflection |
| Scan | |
| Scan range: | 3.000 - 40.000 ° (2θ) |
| Step size: | 0.01 ° (2θ) |
| Speed: | 10.0 °/min (2θ) |
| Scan mode: | Continuous |
| Used wavelength | |
| Intended wavelength type: | Ka1 |

(continued)

| | |
|---|---|
| Used wavelength | |
| Kα1: | 1.540598 Å |
| Kα2: | 1.544426 Å |
| Kα2/Kα1 intensity ratio: | 0.50 |
| Kα: | 1.541874 Å |
| Kα: | 1.392250 Å |
| Instrument Details | |
| X-Ray Generator | |
| Tube output voltage: | 40 kV |
| Tube output: | 15 mA |
| High-voltage generation method: | High-frequency Cockcroft-Walton method |
| Stability: | Within ±0.05% for both the tube voltage and tube current, with reference to ±10% of input power variation. |
| X-ray tube | |
| Name: | Toshiba Analix type A-26L |
| Anode material: | Cu |
| Maximus output: | 0.60 kW |
| Focus size: | 1 x 10 mm |
| Kβ Filter | |
| Name: | Ni-filter |
| Thickness (mm): | 0.015 |
| Material: | Ni |
| Goniometer (Angle measuring device) | |
| Type: | Vertical θ/2θ |
| Goniometer radius: | 150 mm |
| Scanning axis: | θ/2θ linked |
| 2θ scanning range: | +2 ° to +140 ° |
| θ/2θ axis minimum step angle: | 0.005 ° (2θ) |
| Position speed: | 500 °/min (2θ) |
| Scanning speed: | 0.01 to 100 °/min |
| Datum angle: | 2θ = 10° |
| X-ray take-off angle: | 6 ° (fixed) |
| Slit | |
| DS: | 1.25° |
| IHS: | 10.0 mm |
| SS: | none (open) |
| RS: | none (open) |
| Incident side Soller slit: | 2.5° |
| Receiving side Soller slit: | 2.5 ° |

(continued)

| Detector | |
|---|---|
| Name: | D/teX Ultra High-speed 1D Detector |
| Window material: | Be |
| Effective window size: | 13 mm (H) x 20 mm (W) |
| Dimensions: | 80 mm (L) |

The Powder X-Ray diffractograms of Flurbiprofen-Lysine-Gabapentin 1:1:1 and Ibuprofen-Lysine-Gabapentin 1:1:1 co-crystals are reported in Figure 1.

The XRPD peak lists of the co-crystals are reported in Table 2 and in Table 3 below:

Table 2. XRPD Peak List of Flurbiprofen-Lysine-Gabapentin co-crystal

| Position 2 θ (°) | d-spacing [Å] | Intensity |
|---|---|---|
| 6.88 | 12.84706 | 780 |
| 9.35 | 9.44693 | 22400 |
| 10.38 | 8.51275 | 2300 |
| 10.92 | 8.09719 | 511 |
| 12.24 | 7.22378 | 248 |
| 15.20 | 5.82425 | 3180 |
| 16.01 | 5.51406 | 1530 |
| 17.21 | 5.1483 | 5850 |
| 18.30 | 4.84484 | 19300 |
| 18.83 | 4.70847 | 7640 |
| 19.74 | 4.49477 | 6520 |
| 20.66 | 4.29526 | 6340 |
| 21.93 | 4.04977 | 2010 |
| 24.03 | 3.69988 | 2610 |
| 24.77 | 3.5922 | 2290 |
| 25.53 | 3.4861 | 442 |
| 26.32 | 3.383 | 425 |
| 27.86 | 3.1998 | 1650 |
| 28.95 | 3.08212 | 1870 |
| 29.78 | 2.99748 | 652 |
| 31.52 | 2.83627 | 248 |
| 32.99 | 2.71279 | 627 |
| 34.06 | 2.63014 | 307 |
| 35.93 | 2.49756 | 410 |
| 37.53 | 2.39471 | 424 |
| 39.18 | 2.29746 | 153 |
| 40.81 | 2.20918 | 330 |

Table 3. XRPD Peak List of Ibuprofen-Lysine-Gabapentin co-crystal

| Position 2 θ (°) | d-spacing [Å] | Intensity |
|---|---|---|
| 6.86 | 12.87176 | 666 |
| 9.45 | 9.35542 | 34800 |
| 10.27 | 8.61064 | 1970 |
| 11.98 | 7.37902 | 249 |
| 14.72 | 6.01471 | 1010 |
| 15.85 | 5.58853 | 2280 |
| 17.14 | 5.16973 | 16100 |
| 17.61 | 5.0328 | 9120 |
| 18.70 | 4.74233 | 14500 |
| 20.03 | 4.42983 | 10600 |
| 22.31 | 3.98153 | 1960 |
| 24.13 | 3.68462 | 2200 |
| 25.06 | 3.55052 | 2510 |
| 25.57 | 3.48078 | 2430 |
| 26.29 | 3.38739 | 900 |
| 27.87 | 3.19912 | 2010 |
| 28.56 | 3.12317 | 2920 |
| 30.62 | 2.91753 | 280 |
| 31.13 | 2.87064 | 284 |
| 32.30 | 2.76943 | 567 |
| 33.09 | 2.70495 | 251 |
| 34.52 | 2.59653 | 570 |
| 35.36 | 2.53672 | 642 |
| 36.57 | 2.455 | 791 |
| 38.56 | 2.33313 | 523 |
| 39.03 | 2.30589 | 400 |
| 41.11 | 2.19415 | 530 |
| 48.93 | 1.85987 | 109 |

XRPD diffractograms of Flurbiprofen-Lysine-Gabapentin and Ibuprofen-Lysine-Gabapentin were very similar, with the reflexes of the respective main peaks in the region between 9 - 10 degrees 2-theta, 15 - 25 degrees 2-theta and 27 - 28 degree 2-theta having similar intensities (see Figure 1 and Tables 2 and 3). This similarity was taken as clear evidence of isostructurality of the co-crystals.

3. Thermal analyses

DSC Analysis

The analysis was carried out using the instrument DSC Mettler Toledo DSC1.

The sample was weighed in an aluminum pan hermetically sealed with an aluminum cover. The analysis was

performed by heating the sample from 25°C to 320°C at 10K/min, under the conditions shown in Table 3a below:

Table 3a

| Temperature Data | |
|---|---|
| Temperature range | -40 °C to 450 °C |
| Temperature accuracy | ± 0.2 K |
| Temperature precision | ± 0.02 K |
| Furnace temperature resolution | ± 0.00006 K |
| Heating rate | 0.02 to 300 K/min |
| Cooling rate | 0.02 to 50 K/min |
| Cooling time | 5 min (100 °C to 0 °C) |
| Calorimetric Data | |
| Sensor type | FRS5 |
| Sensor material | Ceram ic |
| Number of thermocouples | 56 |
| Signal time constant | 1.8 s |
| Indium peak (height to width) | 17 |
| TAWN resolution | 0.12 |
| Sensitivity | 11.9 |
| Resolution | 0.04 μW |
| Digital resolution | 16.8 million points |

The analysis was carried out on samples of Flurbiprofen-Lysine-Gabapentin co-crystal (Figure 2) and Ibuprofen-Lysine-Gabapentin (Figure 3).

The DSC profile of Flurbiprofen-Lysine-Gabapentin showed an endothermic event at 163.48 °C (onset 161.59 °C, Delta H = 153.58 J/g) that was associated with sample melting and degradation (Figure 2). The DSC profile of Ibuprofen-Lysine-Gabapentin showed endothermic event at 165.60 °C (onset 161.60 °C, Delta H = 157.73 J/g), while above 120 °C, multiple partially overlapped endothermic peaks were detectable due to degradation steps (Figure 3). Surprisingly, Flurbiprofen-Lysine-Gabapentin and Ibuprofen-Lysine-Gabapentin showed similar enthalpy of fusion (153.58 and 157.73 J/g) that further support their isostructural packing. Furthermore, lower melting endotherms of co-crystals are supposed to improve the solubility/dissolution profile of the native drug [(a) M. K. Mishra, P. Sanphui, U. Ramamurty and G. R. Desiraju, Cryst. Growth Des., 2014, 14, 3054-3061; (b) P. Sanphui, N. R. Goud, U. B. R. Khandavilli and A. Nangia, Cryst. Growth Des., 2011, 11, 4135-4145].

<u>Thermogravimetric Analysis TGA</u>

The analysis was carried out using the instrument Mettler Toledo TGA/DSC1.

The sample was weighed in an aluminum pan hermetically sealed with an aluminum pierced cover. The analysis was performed by heating the sample from 25°C to 320°C at 10°/min, under the conditions shown in Table 4 below:

Table 4

| Temperature Data | |
|---|---|
| Temperature range | RT to 1100 °C |
| Temperature accuracy | ±1 K |
| Temperature precision | ±0.4 K |
| Heating rate | 0.02 to 250 K/min |

(continued)

| Temperature Data | |
|---|---|
| Cooling time | 20 min (1100 to 100 °C) |
| Sample volume | ≤100 μL |
| Special modes | |
| Automation | 34 sample positions |
| TGA-FTIR | coupled with Thermo Nicolet iS10 spectrometer |
| Balance data | XP5 |
| Measurement range | ≤5 g |
| Resolution | 1.0 μg |
| Weighing accuracy | 0.005% |
| Weighing precision | 0.0025% |
| Internal ring weights | 2 |
| Blank curve reproducibility | better than ±10 μg over the whole temperature range |

The TGA experiment of Flurbiprofen-Lysine-Gabapentin showed a weight reduction of only 1.686 % before the melting point, confirming that the co-crystal was almost free from solvents (Figures 4).

The TGA analysis of Ibuprofen-Lysine-Gabapentin showed a loss of 8.007% by weight confirming the loss of solvation water (Figures 5).

4. FT-Raman and FT-IR

FT-Raman

Raman spectra were recorded with a Nicolet iS50 FT-IR Spectrometer. The excitation source was an Nd-YAG laser (1064 nm) in the backscattering (180°) configuration. The focused laser beam diameter was approx. 50 mm and the spectral resolution 4 $cm^{-1}$. The spectra were recorded with a laser power at the sample of approx. 100 mW.

FT-IR

The analysis was carried out using an instrument Thermo Nicolet iS50 - ATR module Spectrometer equipped with Smart Performer Diamond, DTGS KBr Detector, IR Source, KBr Beam splitter, under the conditions shown in Table 5 below:

Table 5

| Data Collection Information | |
|---|---|
| Number of sample scans: | 32 |
| Number of background scans: | 32 |
| Collection length: | 47.29 sec |
| Resolution: | 4.000 |
| Levels of zero filling: | 2 |
| Number of scan points: | 16672 |
| Number of FFT points: | 65536 |
| Laser frequency: | 15798.3 cm-1 |
| Interferogram peak position: | 8192 |
| Apodization: | N-B strong |

(continued)

| Data Collection Information | |
|---|---|
| Phase correction: | Mertz |
| Number of background scans: | 32 |
| Background gain: | 1.0 |
| Sample gain: | 8 |
| Aperture | 100 |
| Optical velocity | 0.6329 |

The Raman spectra of Flurbiprofen-Lysine-Gabapentin and Ibuprofen-Lysine-Gabapentin co-crystal are reported in Figures 6 and 7 and the peaks lists are reported in Tables 6 and 7.

Table 6: Raman peaks list of Flurbiprofen-Lysine-Gabapentin co-crystal

| Wavenumber ($cm^{-1}$) | Rel. Intensity |
|---|---|
| 3069 | 0.002 |
| 2970 | 0.001 |
| 2919 | 0.005 |
| 2861 | 0.001 |
| 2240 | 0.001 |
| 2147 | 0.001 |
| 1624 | 0.016 |
| 1604 | 0.007 |
| 1513 | 0.002 |
| 1450 | 0.004 |
| 1287 | 0.007 |
| 1161 | 0.001 |
| 1038 | 0.002 |
| 1001 | 0.005 |
| 977 | 0.001 |
| 878 | 0.001 |
| 737 | 0.003 |
| 618 | 0.002 |
| 506 | 0.001 |
| 414 | 0.001 |
| 272 | 0.001 |
| 223 | 0.002 |
| 87 | 0.002 |

Table 7: Raman peaks list of Ibuprofen-Lysine-Gabapentin co-crystal

| Wavenumber ($cm^{-1}$) | Rel. Intensity |
|---|---|
| 3055 | 0.001 |
| 2919 | 0.006 |
| 2868 | 0.002 |
| 1613 | 0.003 |
| 1449 | 0.005 |
| 1396 | 0.001 |
| 1344 | 0.001 |
| 1187 | 0.003 |
| 1119 | 0.001 |
| 1091 | 0.001 |
| 979 | 0.001 |
| 885 | 0.001 |
| 835 | 0.002 |
| 757 | 0.001 |
| 704 | 0.001 |
| 640 | 0.001 |
| 507 | 0.001 |
| 414 | 0.001 |
| 63 | 0.011 |

The FT-IR spectra of Flurbiprofen-Lysine-Gabapentin and Ibuprofen-Lysine-Gabapentin co-crystals are shown in Figures 8 and 9 and the peaks lists are reported in Tables 8 and 9.

Table 8: FT-IR peaks list of Flurbiprofen-Lysine-Gabapentin co-crystal

| Wavenumber ($cm^{-1}$) | Rel. Intensity | Wavenumber ($cm^{-1}$) | Rel. Intensity |
|---|---|---|---|
| 2965 | 0.011 | 10612 | 0.089 |
| 2932 | 0.257 | 1028 | 0.030 |
| 2849 | 0.026 | 1011 | 0.058 |
| 2763 | 0.017 | 974 | 0.055 |
| 2596 | 0.024 | 944 | 0.044 |
| 2164 | 0.051 | 927 | 0.167 |
| 1628 | 0.189 | 911 | 0.036 |
| 1581 | 0.043 | 873 | 0.202 |
| 1546 | 0.039 | 835 | 0.108 |
| 1519 | 0.013 | 811 | 0.076 |
| 1504 | 0.343 | 793 | 0.017 |
| 1475 | 0.035 | 765 | 0.231 |
| 1451 | 0.063 | 737 | 0.086 |
| 1413 | 0.070 | 715 | 0.135 |

(continued)

| Wavenumber (cm$^{-1}$) | Rel. Intensity | Wavenumber (cm$^{-1}$) | Rel. Intensity |
|---|---|---|---|
| 1392 | 0.542 | 697 | 0.388 |
| 1359 | 0.094 | 661 | 0.043 |
| 1340 | 0.020 | 644 | 0.102 |
| 1282 | 0.182 | 619 | 0.100 |
| 1240 | 0.022 | 578 | 0.073 |
| 1232 | 0.079 | 557 | 0.159 |
| 1206 | 0.059 | 522 | 0.077 |
| 1179 | 0.022 | 507 | 0.109 |
| 1156 | 0.098 | 478 | 0.046 |
| 1129 | 0.041 | 450 | 0.034 |
| 1112 | 0.025 | 421 | 0.349 |
| 1089 | 0.065 | 379 | 0.018 |

Table 9: FT-IR peak list of Ibuprofen-Lysine-Gabapentin co-crystal

| Wavenumber (cm$^{-1}$) | Rel. Intensity | Wavenumber (cm$^{-1}$) | Rel. Intensity |
|---|---|---|---|
| 2924 | 0.146 | 1090 | 0.040 |
| 2865 | 0.014 | 1063 | 0.023 |
| 2596 | 0.011 | 1025 | 0.028 |
| 2187 | 0.004 | 1002 | 0.011 |
| 2162 | 0.023 | 975 | 0.023 |
| 1900 | 0.006 | 945 | 0.036 |
| 1854 | 0.005 | 929 | 0.008 |
| 1816 | 0.004 | 910 | 0.020 |
| 1787 | 0.006 | 881 | 0.080 |
| 1765 | 0.006 | 848 | 0.037 |
| 1738 | 0.004 | 819 | 0.006 |
| 1725 | 0.010 | 784 | 0.040 |
| 1710 | 0.009 | 743 | 0.062 |
| 1691 | 0.007 | 715 | 0.140 |
| 1631 | 0.109 | 683 | 0.032 |
| 1581 | 0.016 | 633 | 0.009 |
| 1547 | 0.028 | 618 | 0.034 |
| 1502 | 0.194 | 593 | 0.011 |
| 1475 | 0.027 | 579 | 0.018 |
| 1451 | 0.036 | 556 | 0.082 |
| 1411 | 0.016 | 506 | 0.031 |
| 1392 | 0.283 | 495 | 0.007 |

(continued)

| Wavenumber ($cm^{-1}$) | Rel. Intensity | Wavenumber ($cm^{-1}$) | Rel. Intensity |
|---|---|---|---|
| 1360 | 0.061 | 422 | 0.173 |
| 1287 | 0.062 | 399 | 0.029 |
| 1242 | 0.029 | 376 | 0.022 |
| 1206 | 0.018 | | |
| 1183 | 0.006 | | |
| 1160 | 0.042 | | |
| 1114 | 0.014 | | |

Even in the Raman and FT-IR spectra, significant similarities among the co-crystals were observed, together with some differences that could be due to the presence of different NSAIDs. All the spectra seemed to indicate that the two crystalline systems are characterized by a very similar local environment, especially regarding Lysine and Gabapentin, and show comparable vibrational behaviors.

5. Liquid and solid state NMR

$^{1}H$-Nuclear magnetic resonance (NMR) spectra were recorded in the indicated solvent with tetramethylsilane (TMS) as internal standard on a Bruker Avance3 400 MHz instrument. Chemical shifts are reported in parts per million (ppm) relative to the internal standard. Abbreviations are used as follows: s=singlet, d= doublet, t=triplet, q=quartet, m=multiplet, dd=doublets of doublet, br=broad. Coupling constants (J values) are given in hertz (Hz).

The solid-state $^{13}C$ CPMAS spectra of the samples were acquired with a Jeol ECZR 600 instrument, operating at 600.17 and 150.91 MHz, respectively for $^{1}H$ and $^{13}C$ nuclei. The powder samples were packed into a cylindrical zirconia rotor with a 3.2 mm o.d. and a 60 μl volume. A sample was collected from each batch and used without further preparations to fill the rotor. The $^{13}C$ CPMAS spectra were acquired at room temperature, at a spinning speed of 20 kHz, using a ramp cross-polarization pulse sequence with a 90° $^{1}H$ pulse of 2.1 μs and a contact time of 3.5 ms. An optimized recycle delay from 5.7 for co-crystal to 100 s was used, for a number of scans from 2200 to 20. For every spectrum, a two-pulse phase modulation (TPPM) decoupling scheme was used, with a radiofrequency field of 108.5 kHz. The $^{13}C$ chemical shift scale was calibrated through the methylene signal of external standard glycine (at 43.7 ppm). As for the $^{13}C$ $T_1$-$^{1}H$ analysis, 12 spectra were acquired for 350 scans with different relaxation delays, included in the range 0.1-60 s and calculated by the Delta v5.2.1 software through an exponential algorithm. The spectra were acquired at a spinning speed of 20 kHz at room temperature using a ramp cross-polarization pulse sequence with a 90° $^{1}H$ pulse of 2.1 μs and a contact time of 2 ms.

$^{1}H$-NMR spectra of Flurbiprofen-Lysine-Gabapentin

$^{1}H$-NMR spectrum of the co-crystal confirmed the concomitant presence in the sample of the Flurbiprofen-Lysine-Gabapentin with 1:1:1 stoichiometry.

The multiplicity and the assignment of the signals in line with the atoms numbering shown in the scheme 1 are reported in Table 10:

Scheme 1. Chemical structure and atom numbering of Flurbiprofen-Lysine-Gabapentin

Scheme 1

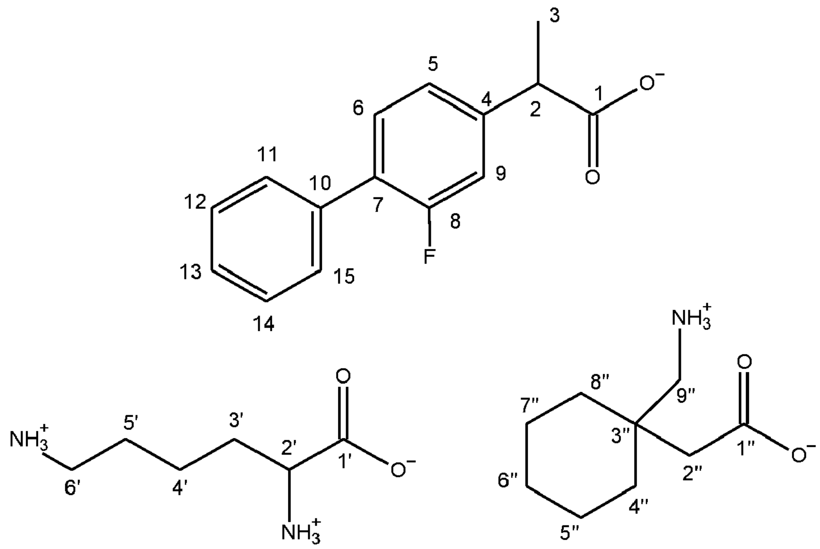

Table 10: $^1$H-NMR

| δ ppm | Multiplicity | Assignment |
|---|---|---|
| 7.62-7.64 | m, 2H | Ar FLU |
| 7.44-7.55 | m, 4H | Ar FLU |
| 7.19-7.25 | m, 2H | Ar FLU |
| 3.69-3.77 | m, 2H | CH (2') LYS; CH (2) FLU |
| 3.00-3.03 | m, 4H | $CH_2$ (6') LYS; $CH_2$ (9") GAB |
| 2.42 | s, 2H | $CH_2$ (2") GAB |
| 1.87-1.93 | m, 2H | $CH_2$ (3') LYS |
| 1.72 | quint., J = 7.6 Hz, 2H | $CH_2$ (5') LYS |
| 1.37 -1.51 | m, 15H | 5 $CH_2$ (4",5",6",7",8") GAB; $CH_2$(4') LYS; $CH_3$ (3) FLU |
| Flurbiprofen-Lysine-Gabapentin Ratio 1:1:1 | | |

The $^1$H-NMR spectrum (400 MHz, $D_2O$) of Flurbiprofen-Lysine-Gabapentin co-crystal is shown in Figure 10.

$^1$H-NMR spectra of Ibuprofen-Lysine-Gabapentin

$^1$H-NMR spectrum of Ibuprofen-Lysine-Gabapentin co-crystal confirmed the concomitant presence in the sample of Ibuprofen-Lysine-Gabapentin with 1:1:1 stoichiometry.

The multiplicity and the assignment of the signals in line with the atoms numbering shown in the Scheme 2 are reported in Table 11:

Scheme 2. Chemical structure and atom numbering of Ibuprofen-Lysine-Gabapentin

Scheme 2

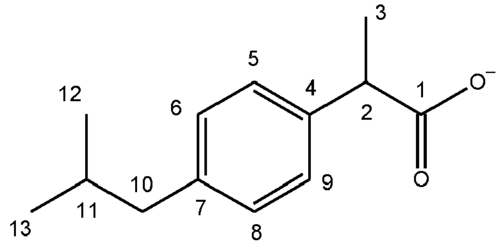

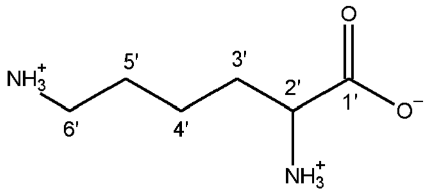

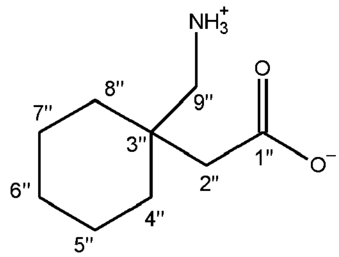

Table 11: $^1$H-NMR

| δ ppm | Multiplicity | Assignment |
|---|---|---|
| 7.21-7.28 | m, 4H | Ar IBU |
| 3.75 | t, J = 6.1 Hz, 1H | CH (2') LYS |
| 3.62 | quart., J = 7.2 Hz, 1H | CH (2) IBU |
| 3.00-3.03 | m, 4H | $CH_2$ (6') LYS; $CH_2$ (9") GAB |
| 2.48 | d, J = 7.1 Hz, 2H | $CH_2$ (10) IBU |
| 2.43 | s, 2H | $CH_2$ (2") GAB |
| 1.81-1.93 | m, 3H | CH (11) IBU; $CH_2$ (3') LYS |
| 1.72 | quint., J = 7.6 Hz, 2H | $CH_2$ (5') LYS |
| 1.37 -1.49 | m, 15H | 5 $CH_2$ (4",5",6",7",8") GAB; $CH_2$ (4') LYS; $CH_3$ (3) IBU |
| 0.88 | d, J = 6.6 Hz, 6H | 2 $CH_3$ (12, 13) IBU |
| Ibuprofen-Lysine-Gabapentin Ratio 1:1:1 | | |

The $^1$H-NMR spectrum (400 MHz, $D_2O$) of Ibuprofen-Lysine-Gabapentin co-crystal is shown in Figure 11.

Solid state $^{13}$C CPMAS spectra of co-crystal Flurbiprofen-Lysine-Gabapentin

A new homogeneous phase of Flurbiprofen-Lysine-Gabapentin was confirmed by $^{13}$C-CPMAS spectra. The stoichiometry was assessed to be 1:1:1, with one independent molecule of Flurbiprofen, Lysine and Gabapentin in the unit cell.

In Table 12 below the characteristic solid state $^{13}$C NMR signals are summarized:

Table 12: solid state $^{13}$C NMR

| $^{13}$C δ (ppm) | Assignment |
|---|---|
| 181.3 | 1 |

(continued)

| $^{13}C$ δ (ppm) | Assignment |
|---|---|
| 179.5 | 1" |
| 178.2 | 1' |
| 160.1 | 8 |
| 148.2 | Aromatic $C_q$ |
| 136.0 | Aromatic $C_q$ |
| 132.3 | Aromatic $C_q$ |
| 130.0 | 2 Aromatic CH |
| 128.3 | 4 Aromatic CH |
| 125.0 | Aromatic CH |
| 113.0 | 9 |
| 54.4 | 2' |
| 49.2 | 9" + 2 |
| 42.6 | 6' |
| 38.6 | 2" + 4" or 8" |
| 34.4 | 3' + 6" + 4" or 8" |
| 30.7 | 5' + 3" |
| 26.1 | 4' |
| 23.1 | 5" or 7" |
| 21.4 | 3 |
| 20.7 | 5" or 7" |

From 13C SSNMR analysis, the carboxylic/carboxylate moieties of the substances are involved in a variety of different interactions (Figure 12 and 13):

- Pure Flurbiprofen (FLU) displays carboxylic homodimers in its crystal structure, leading the associated signal to 183.4 ppm.
- Flurbiprofen sodium salt (NaFLU, Figure 13) displays a carboxylate peak falling at 183.9 ppm;
- In the two Flurbiprofen co-crystals reproduced from literature (salicylamide FLU·SALA and picolinamide, FLU·PICA), the neutral COOH moiety of Flurbiprofen is involved in hydrogen bonds with the aromatic N of the two amides. The corresponding signals resonate at 178.6 (FLU·SALA) and 177.8 (FLU·PICA) ppm respectively.
- Pure Gabapentin (GAB) is a zwitterion, with a COO- moiety resonating at 179.0 ppm; as sodium salt (NaGAB) a neutral NH2 moiety is present instead of a NH3+ group, but it still has a carboxylate group that resonates at 183.4 ppm.
- Zwitterionic L-Lysine (L-LYS) is characterized by a carboxylate signal at 176.7 ppm, while the double hydrochloride salt of DL-lysine (DL-LYS·2HCl) displays a neutral COOH group, whose peak falls at 171.7 ppm.

The spectrum of Flurbiprofen-Lysine-Gabapentin (FLU-LYS-GAB) features three distinct peaks in the carboxylic region, at 181.3, 179.5 and 178.2 ppm. Given the chemical shifts of the starting materials, it is reasonable to assume that they belong to Flurbiprofen, Gabapentin and Lysine, respectively.

15N CPMAS analysis was performed on the co-crystal, as well as on NaGAB. A comparison of their spectra, together with those of L-LYS and GAB, is displayed in Figures 14 and 15.

Flurbiprofen-Lysine-Gabapentin displays three distinct 15N resonances, at 37.6, 30.2 and 21.6 ppm, which confirms the presence of a single independent molecule of Lysine (accounting for 2 signals) and of Gabapentin (accounting for 1) in the unit cell. The chemical shifts suggest that in the co-crystal the α-N and ε-N of Lysine are protonated (i.e. in the form of NH3+) as well as the nitrogen of Gabapentin (Figures 14 and 15).

Solid state $^{13}C$ CPMAS spectra of co-crystal Ibuprofen-Lysine-Gabapentin

A new homogeneous phase of Ibuprofen-Lysine-Gabapentin was confirmed by $^{13}C$-CPMAS spectra. The stoichiometry was assessed to be 1:1:1, with one independent molecule of Ibuprofen, Lysine and Gabapentin in the unit cell. In Table 13 below the characteristic solid state $^{13}C$ NMR signals are summarized:

Table 13: solid state $^{13}C$ NMR

| $^{13}C$ $\delta$ (ppm) | Assignment |
|---|---|
| 182.8 | 1 |
| 181.3 | 1 |
| 179.1 | 1" |
| 178.0 | 1' |
| 143.4 | Aromatic $C_q$ |
| 142.9 | Aromatic $C_q$ |
| 141.5 | Aromatic $C_q$ |
| 139.9 | Aromatic $C_q$ + CH |
| 129.0 | Aromatic CH |
| 127.6 | Aromatic CH |
| 54.3 | 2' |
| 49.6 | 9" or 2 |
| 49.2 | 9" or 2 |
| 46.1 | 10 |
| 42.4 | 6' |
| 38.5 | 2" + 4" or 8" |
| 34.3 | 3' + 6" + 4" or 8" |
| 30.4 | 5' + 3" + 11 |
| 29.8 | 5" or 7" |
| 25.9 | 4' |
| 23.1 | 5" or 7" + 12 +13 |
| 16.4 | 3 |

Figure 16 displays the $^{13}C$ CPMAS NMR spectrum of Ibuprofen-Lysine-Gabapentin co-crystal.

Regarding $^{13}C$ CPMAS NMR of Ibuprofen-Lysine-Gabapentin, two signals ascribable to Ibuprofen (IBU) appear, at 182.8 and 181.3 ppm (Figure 17). The first chemical shift is very similar to that of pure Ibuprofen (182.9 ppm), in which the COOH moiety is involved in homodimeric interactions, and to that of the carboxylate group of Ibuprofen sodium salt (NaIBU) at 183.4 ppm. This suggests that it corresponds to a carboxylate moiety, as further implied by the very much lower chemical shift of a neutral COOH of Ibuprofen in the co-crystal with L-proline (176.7 ppm). As for the other three carboxylic signals, all the considerations done for Flurbiprofen-Lysine-Gabapentin apply here as well. The same can be said for the 15N CPMAS spectra, displayed in Figures 18 and 19.

The 13C CPMAS spectra of, Flurbiprofen-Lysine-Gabapentin and Ibuprofen-Lysine-Gabapentin co-crystals are very similar as shown in Figure 20. Many of the signals of the two co-crystals (highlighted in Figure 20 by means of dashed lines) coincide. Careful analysis of the signals points out that all the recurring signals in the aliphatic region (below 80 ppm) correspond to the aliphatic C nuclei of Lysine and Gabapentin. The most apparent differences relate to the aromatic range (110-170 ppm), which is reasonable, since the two different NSAIDs are those containing aromatic carbons. Most notably, the same similarities can be observed in the carboxylic region, with the carboxylic signals of Flurbiprofen-Lysine-Gabapentin appearing also for Ibuprofen-Lysine-Gabapentin.

The same remarkable spectral similarities (highlighted by dashed lines) can be observed in the 15N CPMAS

spectra of the samples (Figure 21).

From 13C and 15N data, the local environment of the Lysine and Gabapentin fragments seems to be the same for the two crystal forms. In all cases, Gabapentin is a zwitterion, while Lysine displays the usual COO- and α-NH3+ groups. The ε-N of Lysine and the COOH group of the NSAID share the carboxylic proton.

The results strongly suggested the occurrence of a protonic transfer from the COOH moiety of Flurbiprofen and Ibuprofen, which turns into a carboxylate moiety, to the only possible acceptor, the ε-NH2 of Lysine.

In line with the solid state 13C and 15N NMR analysis, in the present co-crystals Flurbiprofen and Ibuprofen carboxylic group is deprotonated and interacts with protonated Lysine ε-$NH_3^+$ group through ionic bonds forming a neutral salt. The Flurbiprofen and Ibuprofen Lysine neutral salt interacts with Gabapentin through non-ionic bonds forming a stable co-crystal.

6. In vivo studies

Inflammatory pain induced by carrageenan intraplantar injection in rats

Male Wistar rats (270-280 g) (Envigo, Italy), were housed 2 - 3 per cage under controlled illumination (12:12 h light : dark cycle; light on 06.00 h) and standard environmental conditions (room temperature 22 ± 1 °C, humidity 60 ± 10%) for at least 1 week before experimental use. Rat chow and tap water were available ad libitum. The procedures were approved by the Animal Ethics Committee of University of Campania "Luigi Vanvitelli". Animal care was in compliance with Italian Legislative Decree (D.L. 116/92) and European Commission Directive (O.J. of E.C. L358/1, 18/12/86) regulations on the protection of laboratory animals. All efforts were made to minimize animal suffering and the number of animals used.

Carrageenan-induced Rat Paw Edema test method

Peripheral inflammatory pain was induced in the left hind paw of each animal by a single intraplantar injection of 1% λ-carrageenan (100 μl for each rat in 0.9% NaCl) (Sigma-Aldrich, St. Louis, MO), by using a 30 g insulin syringe, accordingly with previous studies (Hajhashemi V et al., The role of central mechanisms in the anti-inflammatory effect of amitriptyline on carrageenan-induced paw edema in rats. Clinics (Sao Paulo). 2010). Vehicles or drugs were orally administered 1 h before the carrageenan injection. The paw volume was measured by Plethysmometer (Ugo Basile, Varese, Italy) before (0 h) and after injection of carrageenan at different time intervals (1, 3 and 5 h post-carrageenan). Edema was expressed as the mean increase in paw volume (ml) relative to control animal group. The inhibition percentage of edema was calculated by the following equation:

$$\% \text{ inhibition of edema} = [(Vc\text{-}Vt) *100\text{-}100$$

where Vc is the edema volume in the control group and Vt is the edema volume in treated group. After 5 h, the animals were sacrificed with a lethal dose of urethane and paw and stomach were dissected for morphological and biochemical evaluations.

Mechanical allodynia

Mechanical allodynia was assessed using the up and down method (Chaplan, S. R., et al., (1994) Quantitative assessment of tactile allodynia in the rat paw. J. Neurosci. Methods 53, 55-63). All animals were allowed to acclimate for ~30-45 minutes on an elevated mesh platform in an enclosure (Ugo Basile, Italy). Calibrated von Frey filaments for rats (Stoelting, Wood Dale, IL, ranging from 4 g to 100 g bending force) were applied to the midplantar surface of the hind paw for 3-4 s (Nour Elhouda Saidi et al., Unilateral 6-Hydroxydopamine-Lesioned Rat as Relevant Model to Study the Pain Related to Parkinson's Disease, NEUROLOGY AND NEUROBIOLOGY (2019). ISSN 2613-7828). The threshold was captured as the lowest force (g) that evoked scratching or licking of the stimulated hind-paw. Animals were tested at baseline (0 h) before the carrageenan injection and at 1, 3, and 5 h post-carrageenan.

Experimental groups and drugs

Vehicle 1 (torpac capsules filled with Avicel PH101, 1cps) (n=4); Vehicle 2 (ethanol/0.9% saline, 1:19, 100ul) (n=4); Indomethacin (10mg/Kg, 100ul) in Vehicle 2 (n=8); Flurbiprofen (5 mg/Kg, 1 cps) (n=8); Gabapentin (3.51mg/Kg, 1cps) (n=8); Flur+Lys+Gaba mixture (11.50 mg/Kg, 1cps) (n=8); Flur/Lys/Gaba co-crystal (11.50 mg/Kg, 1 cps) (n=8). The weight ratio of Flurbiprofen, Lysine and Gabapentin in the Flur+Lys+Gaba mixture is 1:1:1.

Comparative effect of single oral administration of Flur/Lys/Gaba co-crystal and Flurbiprofen alone on carrageenan-induced paw edema in rats

Single administration of both vehicles (capsules or ethanol/0.9% saline, i.g.) did not affect the increase in the rat left paw thickness induced by carrageenan intra-plantar injection, as compared to the untreated contralateral paw (4.71 ± 0.17 mm, 3 h post-carrageenan). In particular, a time-dependent paw swelling was observed starting from 1 h with a peak of edema at 5 h (9.55 ± 0.47 mm, p<0.0001; n=8) post-carrageenan injection (Figure 22). Single administration of Flurbiprofen (5 mg/Kg), performed 1 h before carrageenan injection, significantly reduced rat paw thickness (6.5 ± 0.28 mm at 5 h post-carr, p=0.028, n=8), as compared to vehicle-treated group (Figure 22, Table 14), with a reduction of paw volume of -32.0 % (Table 15). In Table 14 below reported is indicated the statistic of paw thickness in different group of animals treated with Vehicle, Flurbiprofen alone, Gabapentin, Indomethacin, the Flur+Lys+Gaba mixture or the Flur/Lys/Gaba co-crystal after intra-plantar injection of 1% of carrageenan. Each time point represents the mean ± SEM of 8 rats per group. P<0.05 was considered as statistically significant and calculated by using Two-way ANOVA followed by Tukey's post-hoc test. °°°°p<0.0001 vs Contra, *p<0.05, **p<0.01 and ***p<0.001 vs Vehicle and #p<0.05 and ##p<0.01 *vs* Gaba. In table 14 below, "ns" stands for "non significant".

Table 14: Statistic of paw thickness in different group of animals

| | Vehicle | Flurbiprofen | Gaba | Indomethacin | Flur+Lys+Gaba | Flur/Lys/Gaba |
|---|---|---|---|---|---|---|
| **1h** | °°°° | * | **ns** | ** | * | ** |
| **3h** | °°°° | ** | **ns** | ** | ** | *** ## |
| **5h** | °°°° | ** | * | ** | ** | *** # |

Table 15: volume reduction of paw edema in rats (%)

| | Flurbiprofen | Gaba | Indomethacin | Flur+Lys+Gaba | Flur/Lys/Gaba |
|---|---|---|---|---|---|
| 1h | -18.20 | -1.93 | -17.68 | -17.57 | -21.80 |
| 3h | -31.50 | -16.21 | -35.27 | -29.32 | -39.91 |
| 5h | -32.00 | -17.27 | -40.45 | -35.47 | -42.27 |

Whereas, the administration of Flur/Lys/Gaba (11.5 mg/Kg) co-crystal greatly reduced the paw thickness (5.51 ± 0.42 mm at 5h post-carr; p=0.0003, n=8), with an inhibition percentage of paw volume of -42.27 %, as compared to the vehicle-treated rats (Figure 22, Table 15). Similarly, Indomethacin (10 mg/Kg, i.g.), used as a reference drug in this study, reduced paw thickness in a significant way at all time points of observation (5.69 ± 0.27 mm at 5h post-carr; p=0.0002, n=8) with a reduction in percentage of paw volume of -40.45 %, respect to vehicle-treated group (Figure 22, Table 15). Finally, Gabapentin alone at 3.51 mg/Kg was able to reduce paw swelling only at 5 hours after carrageenan injection (7.45 ± 0.26 mm at 5h post-carr; p=0.0284, n=8) with a volume reduction in percentage of -17.27 % (Figure 22, Table 15).Two-way ANOVA analysis showed a significant effect of treatments (F6, 49 = 57.48, P < 0.0001), of time (F3, 147 = 45.10, P <0.0001), and a significant interaction for factors time × treatment (F18, 147 = 7.042, P <0.0001) was observed.

Comparative effect of single oral administration of Flur/Lys/Gaba co-crystal and Flurbiprofen alone on the mechanical allodynia in a rat carrageenan-induced paw edema model

In another set of experiments, single oral administration (i.g) of Flurbiprofen (5 mg/Kg) and of Flur/Lys/Gaba co-crystal (11.5 mg/Kg, cps) were tested on mechanical allodynia in carrageenan-injected animals as compared to Indomethacin (10 mg/kg), Gabapentin (3.51 mg/Kg) or Vehicles (capsules or ethanol/0.9% saline). In particular, the analysis was performed at baseline (0), before the carrageenan intra-plantar injection, and at 1, 3 and 5 hours after carrageenan by using manual Von Frey filaments. We observed that mechanical withdrawal threshold significantly decreased in the ipsilateral paw starting from 1h post-carrageenan and it was maintained until the end of observation (5h) in vehicle-treated animals (8.25 ± 1.61 g at 3h post-carr, p< 0.0001; n=8), as compared to the baseline (80 ± 7.56 g, time 0) (Figure 23). No changes were observed in the contralateral side of carrageenan injection (85 ± 7.31 g at 3h post-carr). Single oral administration of Flurbiprofen showed a strong anti-inflammatory but not significant anti-allodynic effect after paw carrageenan injection. Indeed, Flurbiprofen was not able to increase paw withdrawal threshold in rats

3 h post-carrageenan injection (44.62 ± 14.43 g, p=0.2802; n=8), as compared to vehicle-injected animals (Figure 23). Moreover, Indomethacin (10 mg/Kg) treated animals showed a non-significant trend in increasing paw thresholds at 3 hours post-carrageenan (36 ± 7.21 g, p=0.0567; n=8). On the contrary, Flur/Lys/Gaba co-crystal induced a reduction of mechanical allodynia up to the end of the experiment, starting from 1h post carrageenan injection, reaching a peak at 3 h post carrageenan injection (85.75 ± 9.86 g, p=0.001; n=8) as compared to vehicle-injected animals (Figure 23). Finally, Gabapentin (3.51 mg/Kg) administration showed a trend in decreasing mechanical allodynia, with a peak-time at 3h post-carrageenan injection (66.5 ± 11 g, p=0.0109; n=8) (Figure 24). Two-way ANOVA analysis showed a significant effect of treatments (F6, 49 = 22.13, P < 0.0001), of time (F3, 147 = 39.29, P <0.0001), and significant interaction for factors time × treatment (F18, 147 =2.30, P =0.0035) was observed. Table 16 below indicates the statistic of paw withdrawal threshold (g) in different groups of animals has been represented. P<0.05 was considered as statistically significant and calculated by using Two-way ANOVA followed by Tukey's post-hoc test. °°°°p<0.0001 vs Contra, *p<0.05, **p<0.01 and ***p<0.001 vs Vehicle and #p<0.05 and ##p<0.01 vs Gaba.

In Table 16 below reported, "ns" stands for "not significant".

Table 16: statistic of paw withdrawal threshold (g)

| | Vehicle | Flurbiprofen | Gaba | Indomethacin | Flur+Ls+Gaba | Flur/Lys/Gaba |
|---|---|---|---|---|---|---|
| 1h | °°°° | ns | * | * | *°°°° # | *## |
| 3h | °°°° | ns | * | ns | * | *** |
| 5h | °°°° | ns | * | ns | * | *** ## |

7. Determination of the exposure parameters in plasma of NSAIDs and Gabapentin after their oral administration as capsules in the rats

Aim of the study was the determination of pharmacokinetic parameters of NSAIDs, in particular Flurbiprofen and Ibuprofen, and Gabapentin in NSAID-Lysine-Gabapentin co-crystal compared to the physical mixture of NSAID, Lysine and Gabapentin.

Sprague Dawley male rat (body weights 310 gr at the time of the treatment) were used in this study. The animals were originally supplied by Harlan, Italy. Once receipt from the supplier, the animals were subjected to health examinations and acceptance. The animals were housed, in a group of three, in cages suitable for the species and were routinely kept in the following environment except for short periods of time where experimental procedures dictated otherwise. The animals were acclimatized to local housing conditions for approximately 5 days.

The animals were housed in a single, exclusive room, air conditioned to provide a minimum of 15 air changes/hour. The environmental controls were set to maintain temperature within the range 22°C and relative humidity within the range 50 to 60% with an approximate 12 hour light and 12 hour dark cycle that is controlled automatically. Food (Mucedola Standard GLP diet) and water were available *ad libitum* throughout the study. All animals were weighed on the day of each treatment. Clinical signs were monitored at regular intervals throughout the study in order to assess any reaction to treatment. Each animal was uniquely identified with a coloured spray on the back before the experiment.

At the end of the study animals were sacrificed by exsanguination under anaesthesia.

The experiment was carried on in agreement with the Italian Law D. L.vo 4 marzo 2014, n. 26.

The experimental protocol consisted in the blood and brain tissue sampling on the animals according to the following Tables 17 and 18 and analysis of samples as described below.

Table 17: Blood Sampling

| | |
|---|---|
| Animals/Time Point | 7 time points |
| Time points | 30 min, 1, 2, 3, 6, 8, and 24h |
| Fasting Requirements | Not required |
| Collection Site | Animals will be exsanguinated from caudal vein |
| Collection tube | Li heparin anticoagulant |
| Target Blood Volume | 70 μL |
| Sample Identification | Label indicating: Study number, animal ID, test item ID, sampling time |
| Sample Requirements | Stored in ice and centrifuged at +4° C, 3000 g for 10 minutes |

(continued)

| | |
|---|---|
| Animals/Time Point | 7 time points |
| Final Sample Storage Conditions | -20°C until bioanalysis |

Table 18: Cmax Flurbiprofen and Gabapentin

| | Cmax (ng/mL) Flurbiprofen | Cmax (ng/mL) Gabapentin |
|---|---|---|
| Co-crystal | 14725±1267 | 3008±246 |
| Mixture | 11495±1088 | 2560±157 |

As depicted in Table 18, following oral administration of Flurbiprofen/Lysine/Gabapentin co-crystal and of the physical mixture of Flurbiprofen, Lysine and Gabapentin Flur+Lys+Gaba, given through one capsule/animal, systemic exposure parameters of Gabapentin were higher and reached the statistical significance (t-test=0.017).

For Flurbiprofen, Cmax was higher for the co-crystal compared to the mixture. The difference reached the statistical significance (t-test=0.023).

Thus, the higher Cmax of the co-crystal compared to the mixture means an improved pharmacokinetic profile that can led to a better pharmacological efficacy.

## Claims

**1.** Co-crystal of a nonsteroidal anti-inflammatory drug (NSAID) belonging to the class of phenylpropionic or phenylacetic acids, Lysine and Gabapentin, providing that said nonsteroidal anti-inflammatory drug is not Ketoprofen.

**2.** The co-crystal as claimed in claim 1 wherein the molar ratio of the components in the co-crystal is 1:1:1.

**3.** The co-crystal as claimed in any one of claims 1 or 2, wherein said nonsteroidal anti-inflammatory drug belonging to the class of phenylpropionic acid derivative is selected from Ibuprofen, Flurbiprofen, Fenoprofen, Indoprofen, Loxoprofen, Pelubiprofen, and Naproxen.

**4.** The co-crystal as claimed in any one of claims 1 or 2, wherein said nonsteroidal anti-inflammatory drug belonging to the class of phenylacetic acid derivative is selected from Diclofenac, Felbinac, Ibufenac, Fenclofenac, Tifurac and Ketorolac.

**5.** The co-crystal as claimed any one of claims 1 to 3, wherein said nonsteroidal anti-inflammatory drug is selected from Flurbiprofen and Ibuprofen.

**6.** The co-crystal as claimed in any one of claims 1 to 5, **characterized by** the following XRPD diffraction peaks: 9.3, 17.1, 18.5, 19.8, 22.1, 24.1, 24.9, 27.9 degrees 2-theta ± 0.2 degrees 2-theta.

**7.** The co-crystal as claimed in claim 1 or 2, in which said NSAID is Flurbiprofen, **characterized by** the following XRPD diffraction peaks: 9.3, 10.4, 15.2, 16.0, 17.2, 18.3, 18.8, 19.7, 20.7, 21.9, 24.0, 24.8, 27.9, and 29.0 degrees 2-theta ± 0.2 degrees 2-theta, preferably further **characterized by** the following XRPD diffraction peaks: 6.9, 10.9, 12.2, 25.5, 26.3, 29.8, 31.5, 33.0, 34.0, 35.9, 37.5, 39.2 and 40.8 degrees 2-theta ± 0.2 degrees 2-theta.

**8.** The co-crystal as claimed in claim 7, further **characterized by** one or more of the following:

a DSC thermogram as reported in Figure 2,
a TGA thermogram as reported in Figure 4,
FT Raman and FT-IR spectra of Figures 6 and 8,
solution $^{1}H$-NMR spectrum of Figure 10 and signals of Table 10,
solid state $^{13}C$ CPMAS spectrum of Figure 12 and signals of Table 12, and/or
15N CPMAS spectrum of Figure 14.

**9.** The co-crystal as claimed in claim 1 or 2, in which said NSAID is Ibuprofen, **characterized by** the following XRPD diffraction peaks: 9.5, 10.3, 15.9, 17.1, 17.6, 18.7, 20.0, 22.3, 24.1, 25.1, 25.6, 27,9 and 28.6 degrees 2-theta $\pm$ 0.2 degrees 2-theta, preferably further **characterized by** the following XRPD diffraction peaks: 6.9, 12.0, 14.7, 26.3, 30.6, 31.1, 32.3, 33.1, 34.5, 35.3, 36.6, 38.6, 39.0, 41.1, and 48.9 degrees 2-theta $\pm$ 0.2 degrees 2-theta.

**10.** The co-crystal as claimed in claim 9, further **characterized by** one or more of the following:

a DSC thermogram as reported in Figure 3,
a TGA thermogram as reported in Figure 5,
FT Raman and FT-IR spectra of Figures 7 and 9,
solution $^{1}$H-NMR spectrum of Figure 11 and signals of Table 11,
solid state $^{13}$C CPMAS spectrum of Figure 16 and signals of Table 13, and/or
15N CPMAS spectrum of Figure 18.

**11.** The co-crystal as claimed in any one of claims 1 to 10, wherein said Lysine is (S,R)-Lysine.

**12.** A pharmaceutical composition comprising a co-crystal as claimed in any one of claims 1 to 11 and a least one pharmaceutically acceptable excipient.

**13.** The co-crystal as claimed in any one of claims 1 to 11 or the pharmaceutical composition as claimed in claim 12 for use as a medicament.

**14.** The co-crystal as claimed in any one of claims 1 to 11 or the pharmaceutical composition as claimed in claim 12 for use in the prevention, reduction or treatment of pain and/or inflammation, preferably of acute or chronic pain.

**15.** The co-crystal or the pharmaceutical composition for use as claimed in claim 14, wherein said pain is neuropathic or inflammatory pain and is selected from headache, toothache, menstrual pain, muscle pain, neuropathic pain, pain associated to neuroinflammation, diabetic neuropathy, cancer pain, osteoarthritis, low back pain, sciatalgia, fibromyalgia, trigeminal neuralgia, post-surgical and post-operative pain, post herpetic neuralgia, rheumatoid arthritis, ankylosing spondylitis, frozen shoulder, phantom limb pain or HIV pain.

**16.** Process for the preparation of a co-crystal as claimed in any one of claims 1 to 11, which comprises:

a) suspending a non-steroidal anti-inflammatory drug (NSAID) belonging to the class of phenylpropionic or phenylacetic acids, Lysine, and Gabapentin in a suitable solvent,
b) dissolving said NSAID, Lysine, and Gabapentin optionally by heating the suspension, possibly under stirring, until a clear solution is obtained,
c) optionally cooling the solution, and/or
d) optionally adding an anti-solvent, thus providing the NSAID, Lysine, and Gabapentin co-crystal.

FIG.1

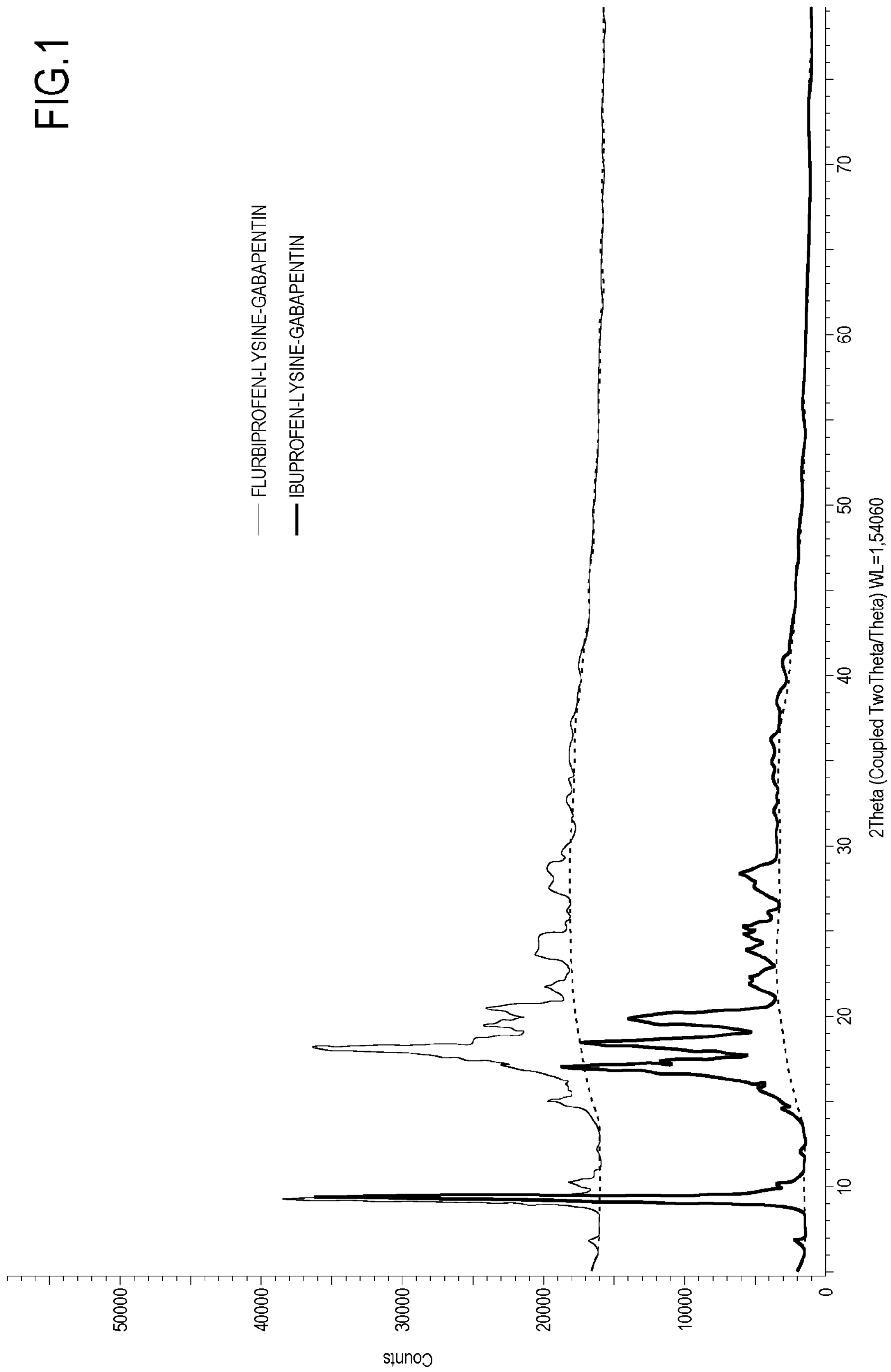
FLURBIPROFEN-LYSINE-GABAPENTIN
IBUPROFEN-LYSINE-GABAPENTIN
Counts
50000
40000
30000
20000
10000
0
10
20
30
40
50
60
70
2Theta (Coupled TwoTheta/Theta) WL=1,54060

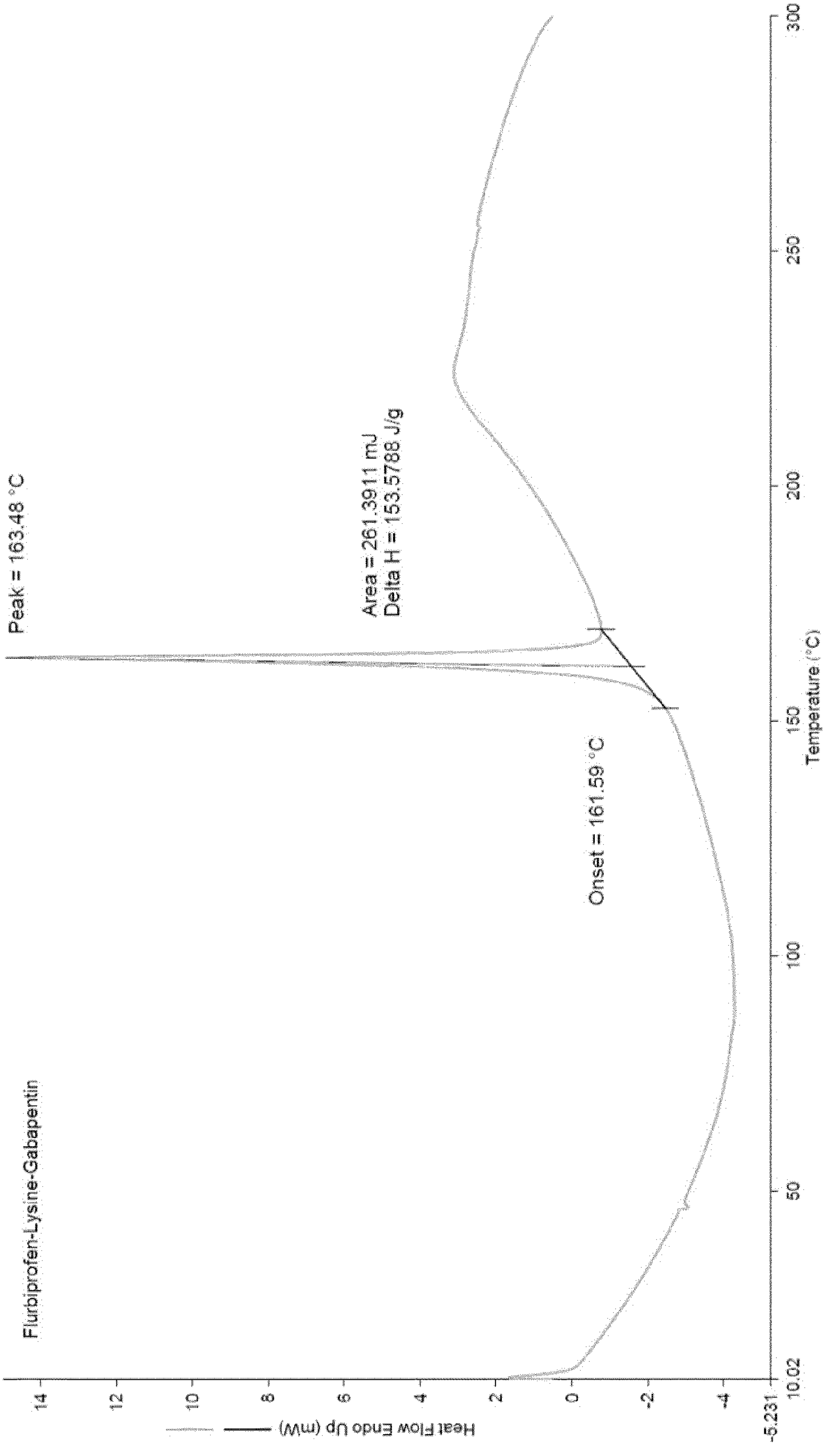
Flurbiprofen-Lysine-Gabapentin
Peak = 163.48 °C
Area = 261.3911 mJ
Delta H = 153.5788 J/g
Onset = 161.59 °C
Heat Flow Endo Up (mW)
Temperature (°C)
14
12
10
8
6
4
2
0
-2
-4
-5.231
10.02
50
100
150
200
250
300

FIG.2

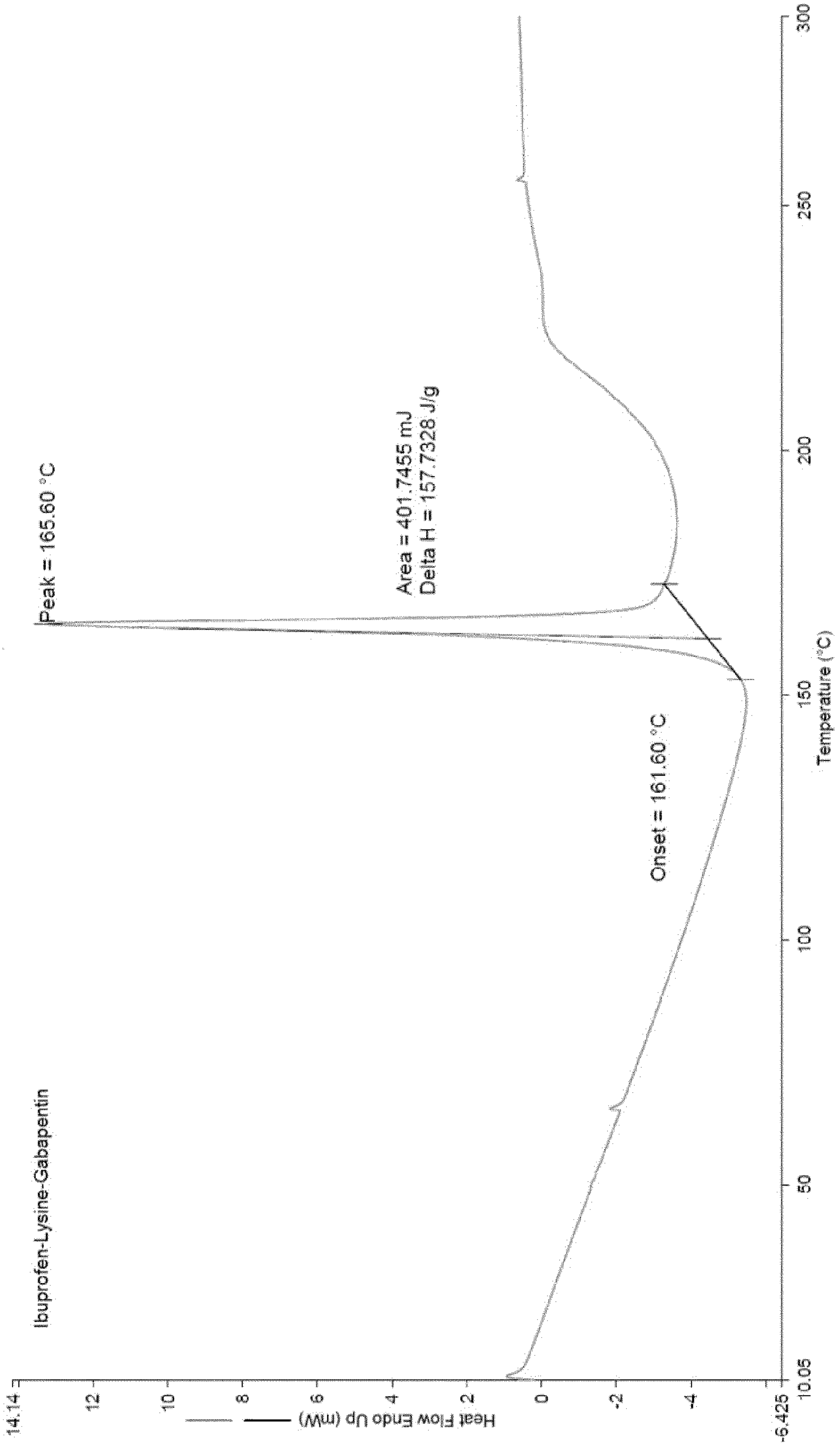
Ibuprofen-Lysine-Gabapentin
Peak = 165.60 °C
Area = 401.7455 mJ
Delta H = 157.7328 J/g
Onset = 161.60 °C
Heat Flow Endo Up (mW)
Temperature (°C)
14.14
12
10
8
6
4
2
0
-2
-4
-6.425
10.05
50
100
150
200
250
300

FIG.3

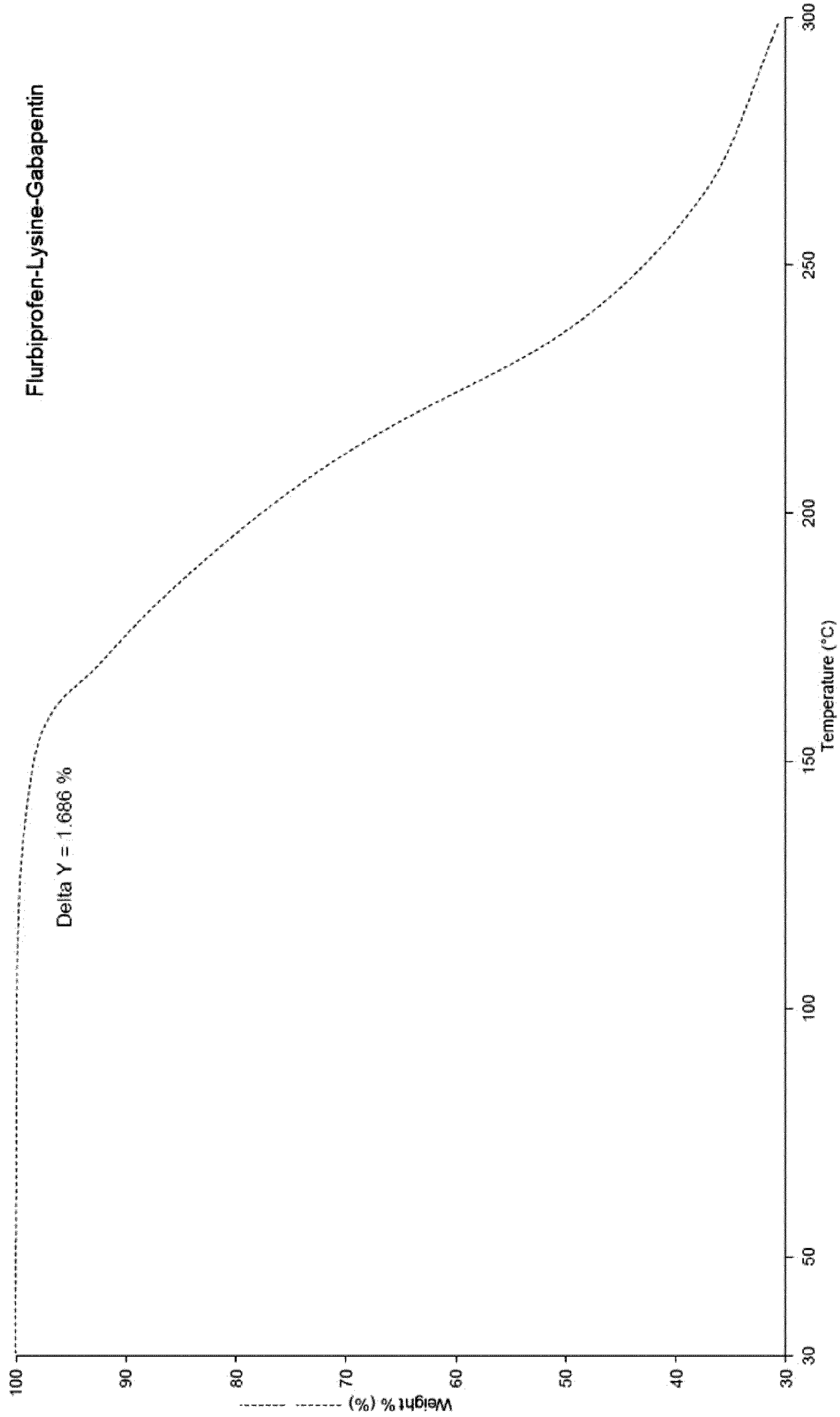
Flurbiprofen-Lysine-Gabapentin
Delta Y = 1.686 %
Weight % (%)
Temperature (°C)
100
90
80
70
60
50
40
30
30
50
100
150
200
250
300

FIG.4

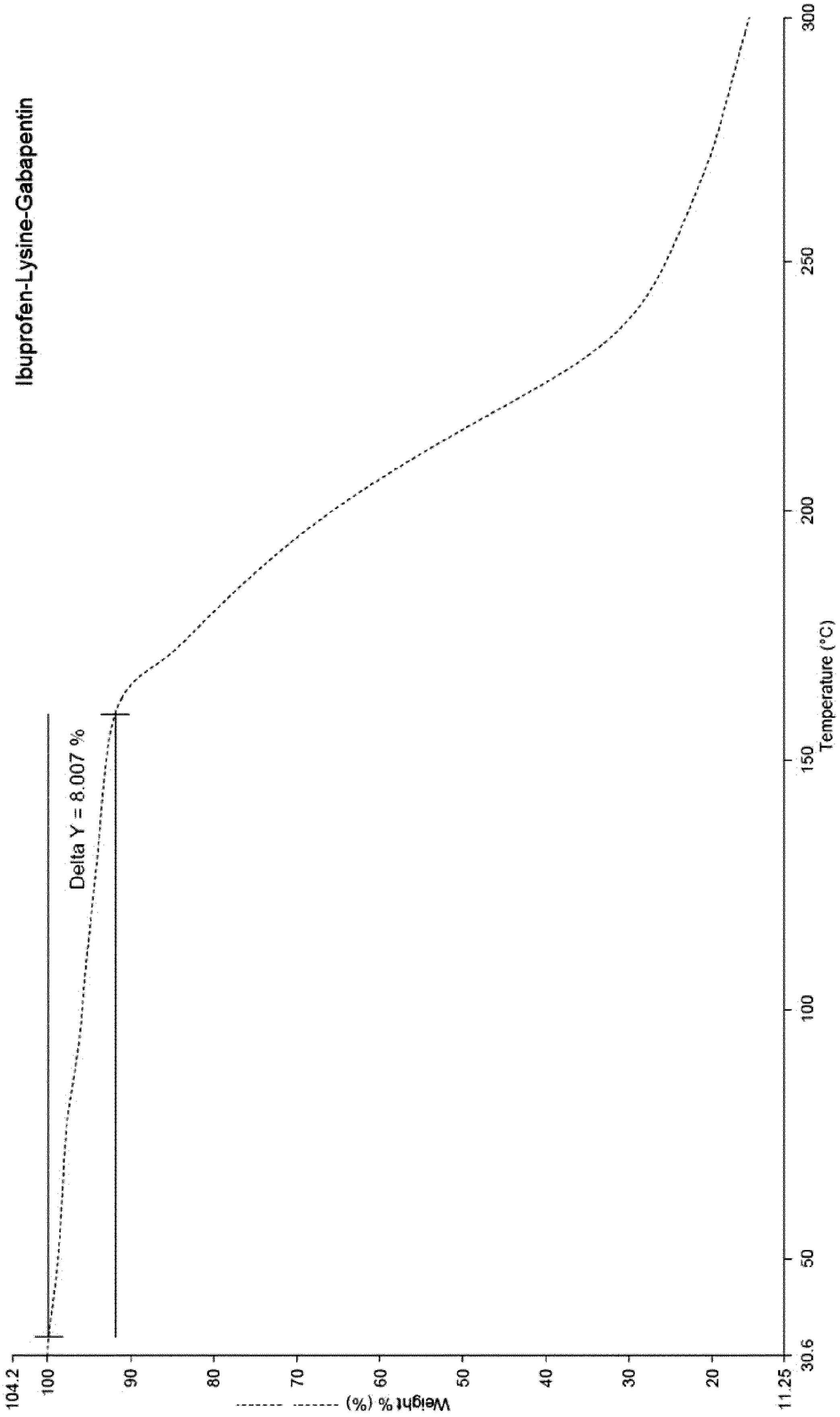
Ibuprofen-Lysine-Gabapentin
Delta Y = 8.007 %
Weight % (%)
104.2
100
90
80
70
60
50
40
30
20
11.25
30.6
50
100
150
200
250
300
Temperature (°C)

FIG.5

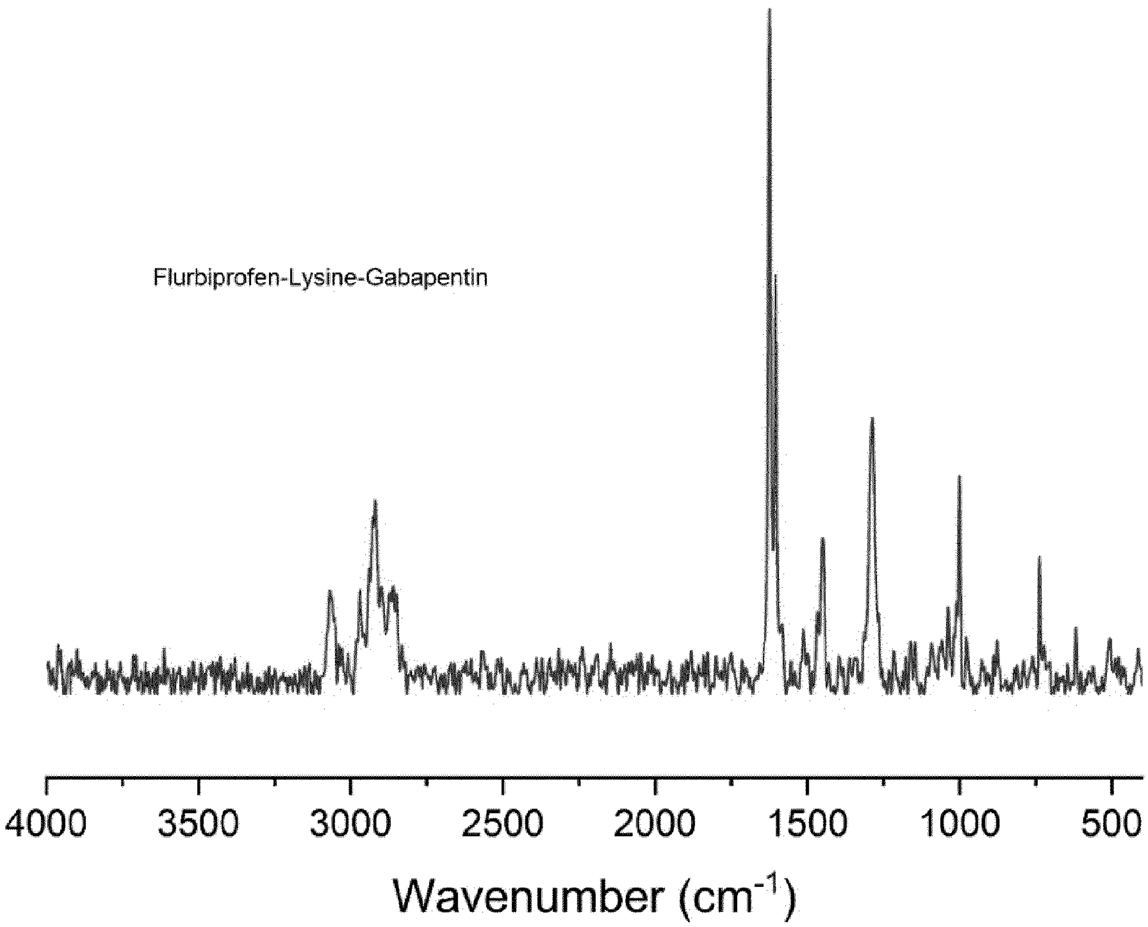
Flurbiprofen-Lysine-Gabapentin
4000
3500
3000
2500
2000
1500
1000
500
Wavenumber (cm-1)

FIG.6

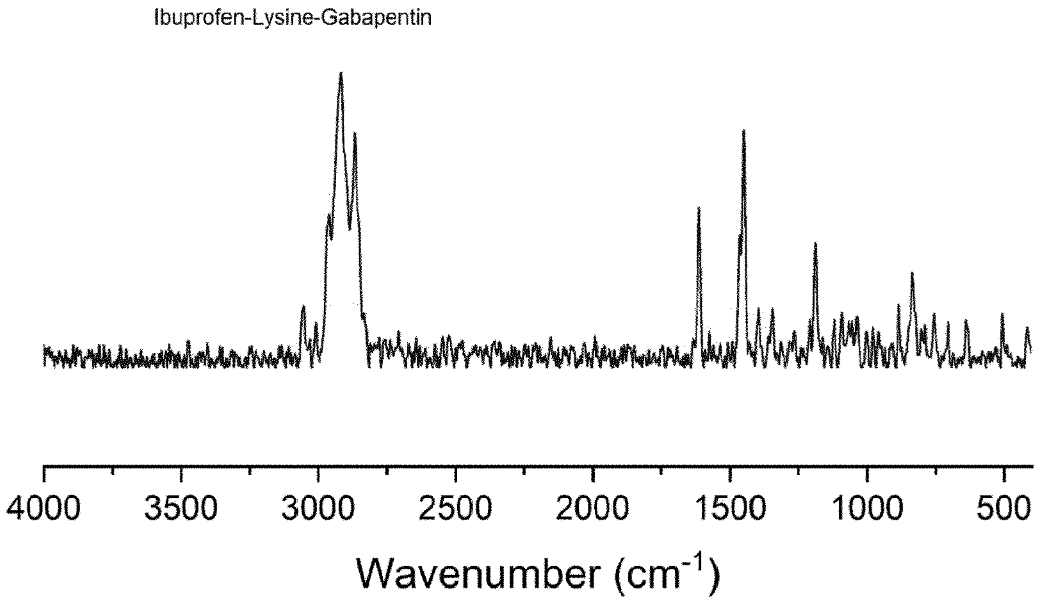
Ibuprofen-Lysine-Gabapentin
4000
3500
3000
2500
2000
1500
1000
500
Wavenumber (cm-1)

FIG.7

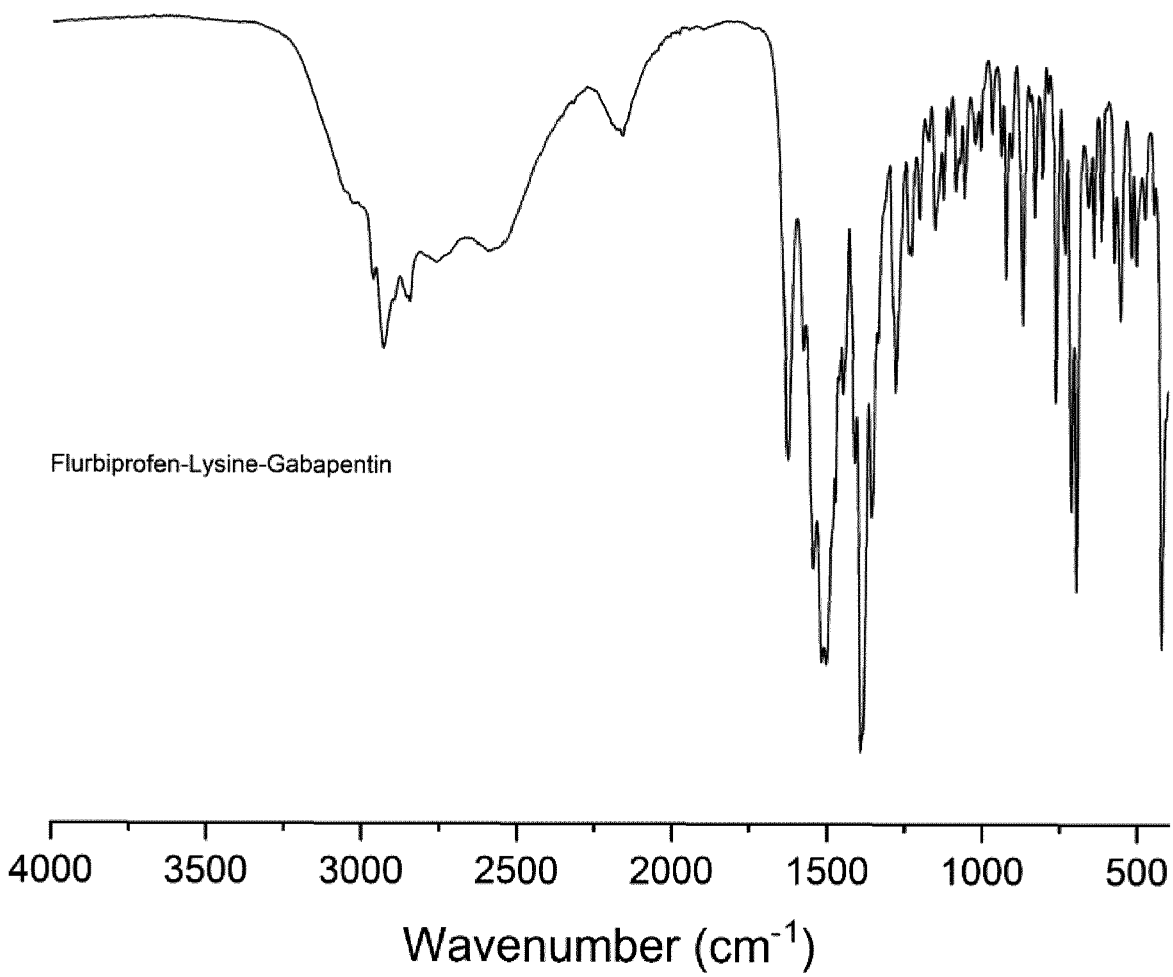
Flurbiprofen-Lysine-Gabapentin
4000
3500
3000
2500
2000
1500
1000
500
Wavenumber (cm-1)

FIG.8

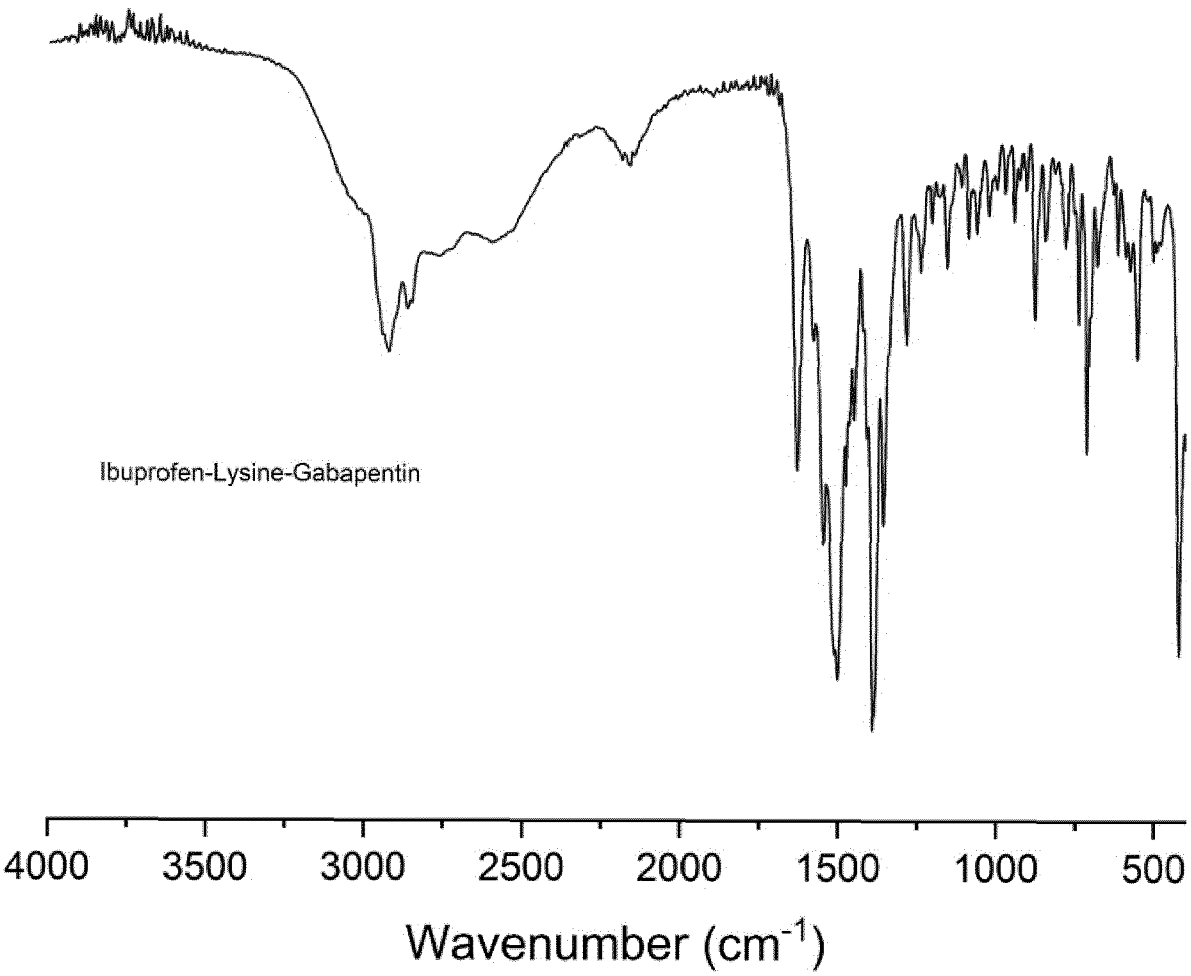
Ibuprofen-Lysine-Gabapentin
4000
3500
3000
2500
2000
1500
1000
500
Wavenumber (cm-1)

FIG.9

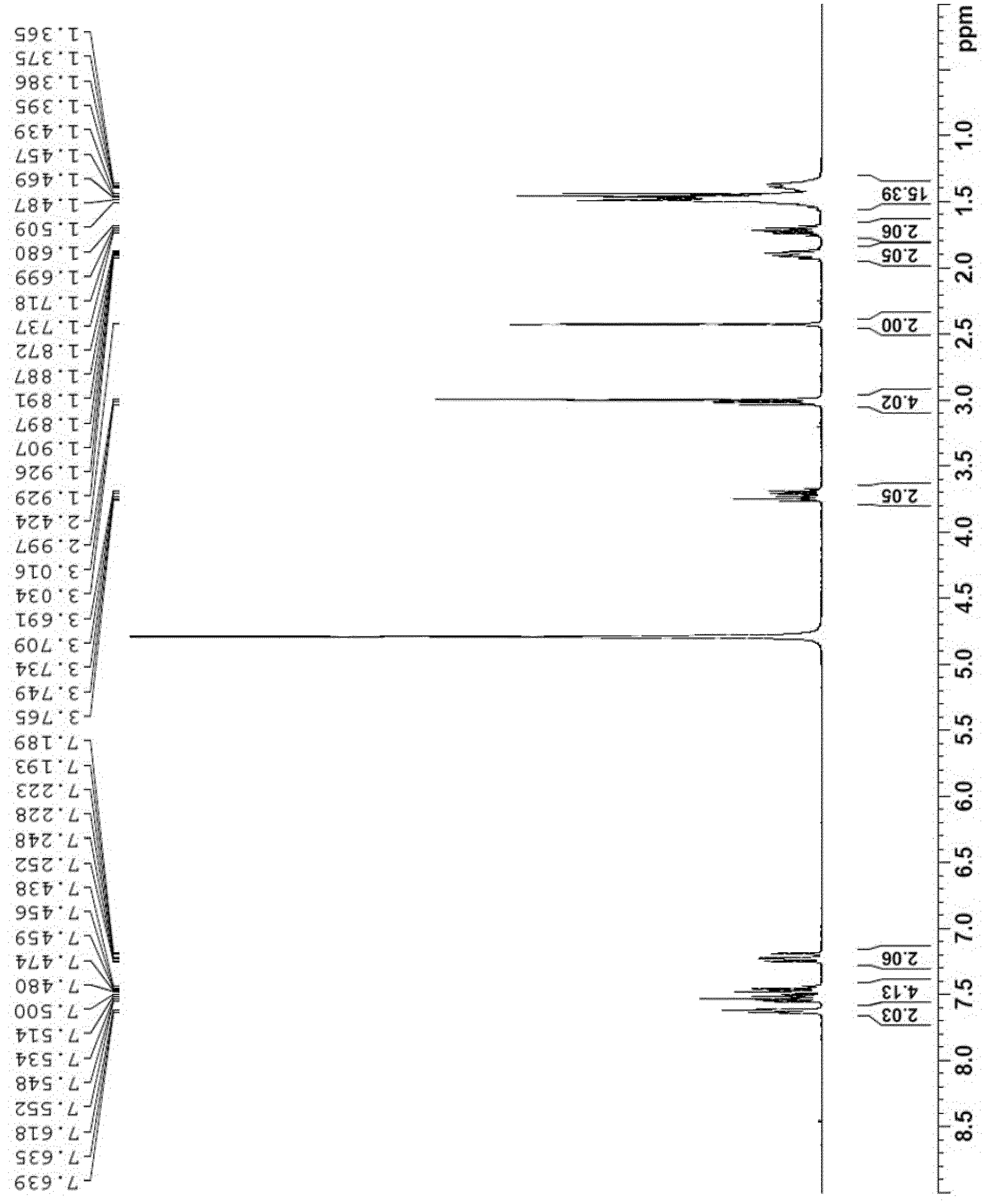
1.365
1.375
1.386
1.395
1.439
1.457
1.469
1.487
1.509
1.680
1.699
1.718
1.737
1.872
1.887
1.891
1.897
1.907
1.926
1.929
2.424
2.997
3.016
3.034
3.691
3.709
3.734
3.749
3.765
7.189
7.193
7.223
7.228
7.248
7.252
7.438
7.456
7.459
7.474
7.480
7.500
7.514
7.534
7.548
7.552
7.618
7.635
7.639
15.39
2.06
2.05
2.00
4.02
2.05
2.06
4.13
2.03
ppm
1.0
1.5
2.0
2.5
3.0
3.5
4.0
4.5
5.0
5.5
6.0
6.5
7.0
7.5
8.0
8.5

FIG.10

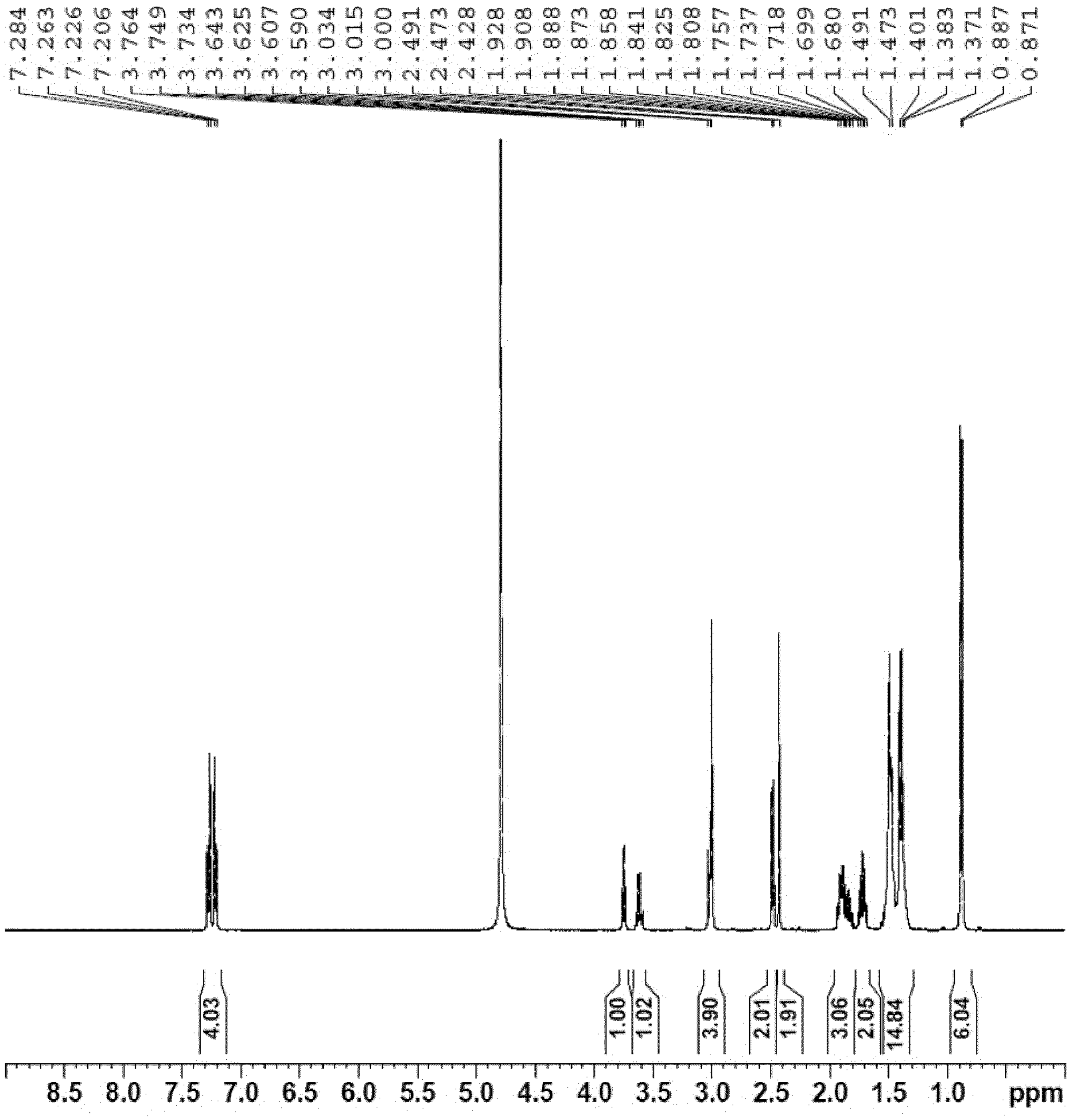
7.284
7.263
7.226
7.206
3.764
3.749
3.734
3.643
3.625
3.607
3.590
3.034
3.015
3.000
2.491
2.473
2.428
1.928
1.908
1.888
1.873
1.858
1.841
1.825
1.808
1.757
1.737
1.718
1.699
1.680
1.491
1.473
1.401
1.383
1.371
0.887
0.871
4.03
1.00
1.02
3.90
2.01
1.91
3.06
2.05
14.84
6.04
8.5
8.0
7.5
7.0
6.5
6.0
5.5
5.0
4.5
4.0
3.5
3.0
2.5
2.0
1.5
1.0
ppm

FIG.11

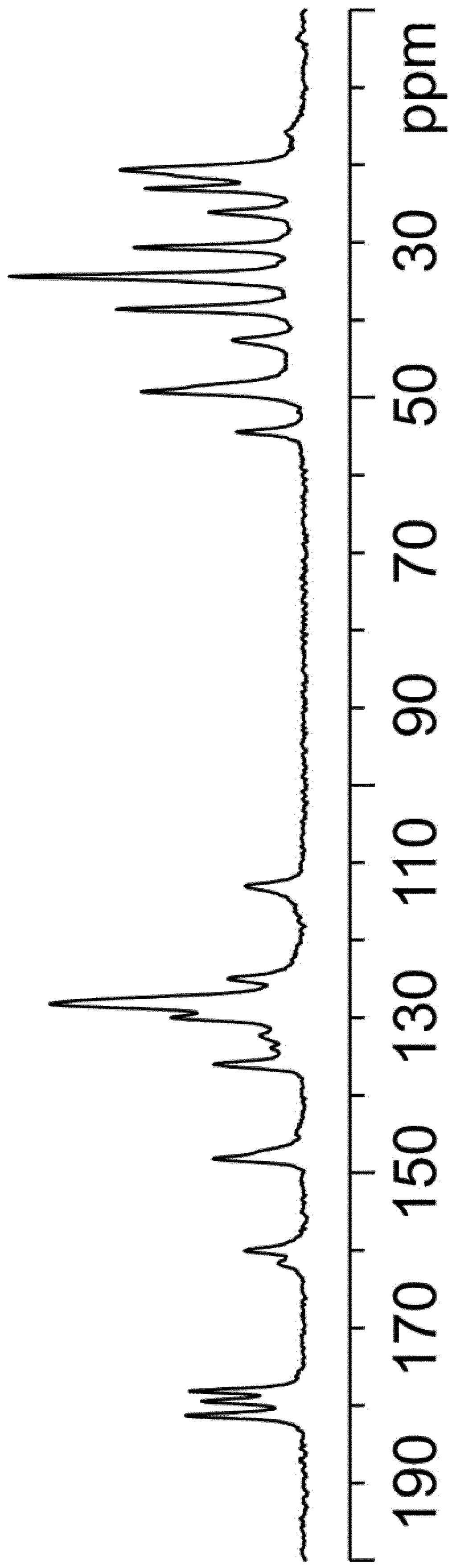


FIG.12

FIG.13

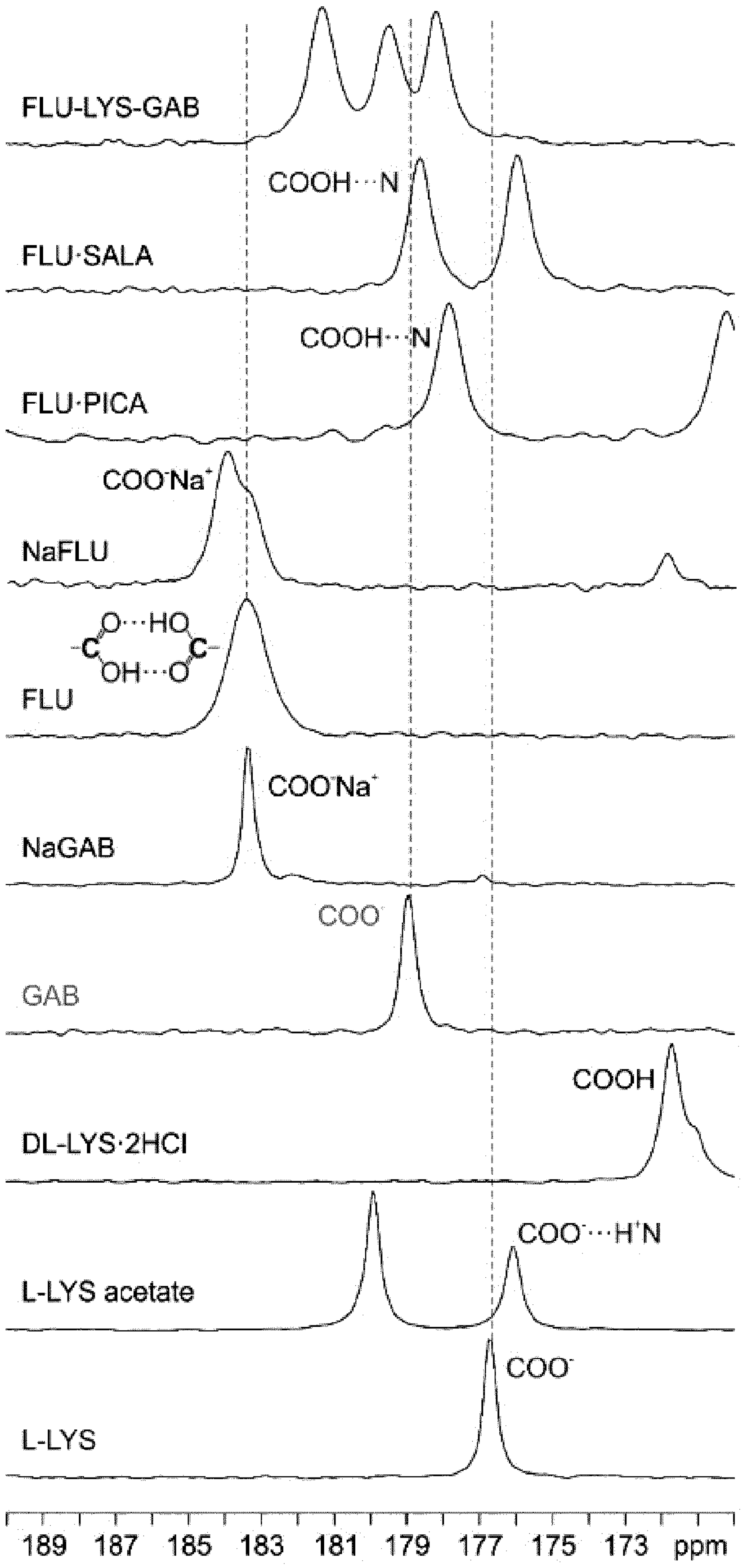
FLU-LYS-GAB
COOH···N
FLU·SALA
COOH···N
FLU·PICA
COO⁻Na⁺
NaFLU
FLU
COO⁻Na⁺
NaGAB
COO⁻
GAB
COOH
DL-LYS·2HCl
COO⁻···H⁺N
L-LYS acetate
COO⁻
L-LYS
189 187 185 183 181 179 177 175 173 ppm

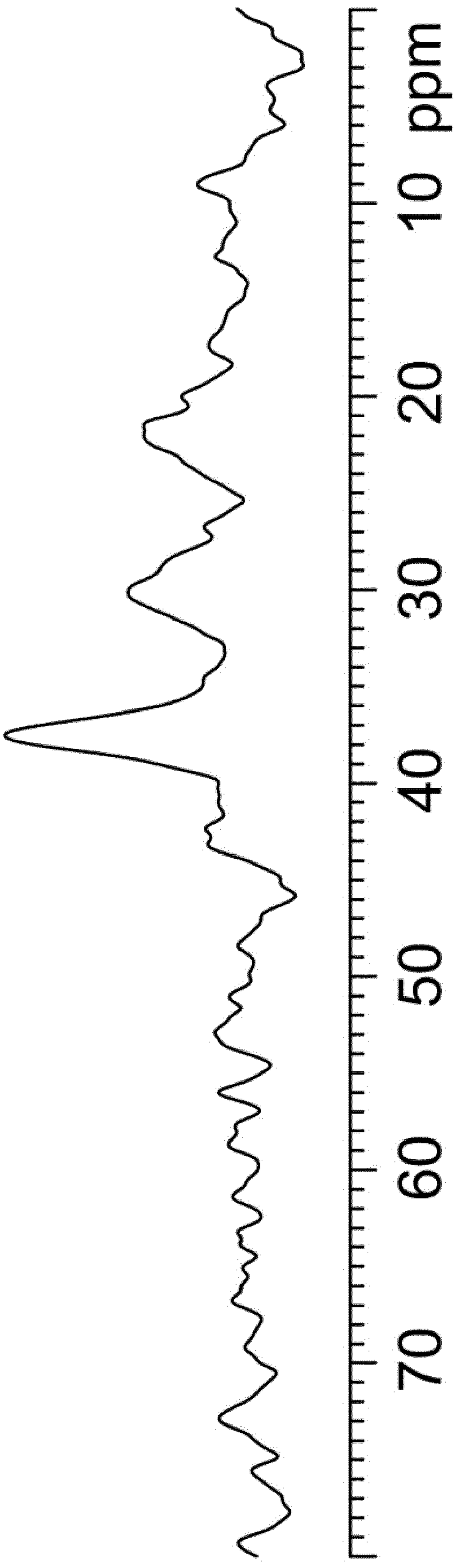


FIG.14

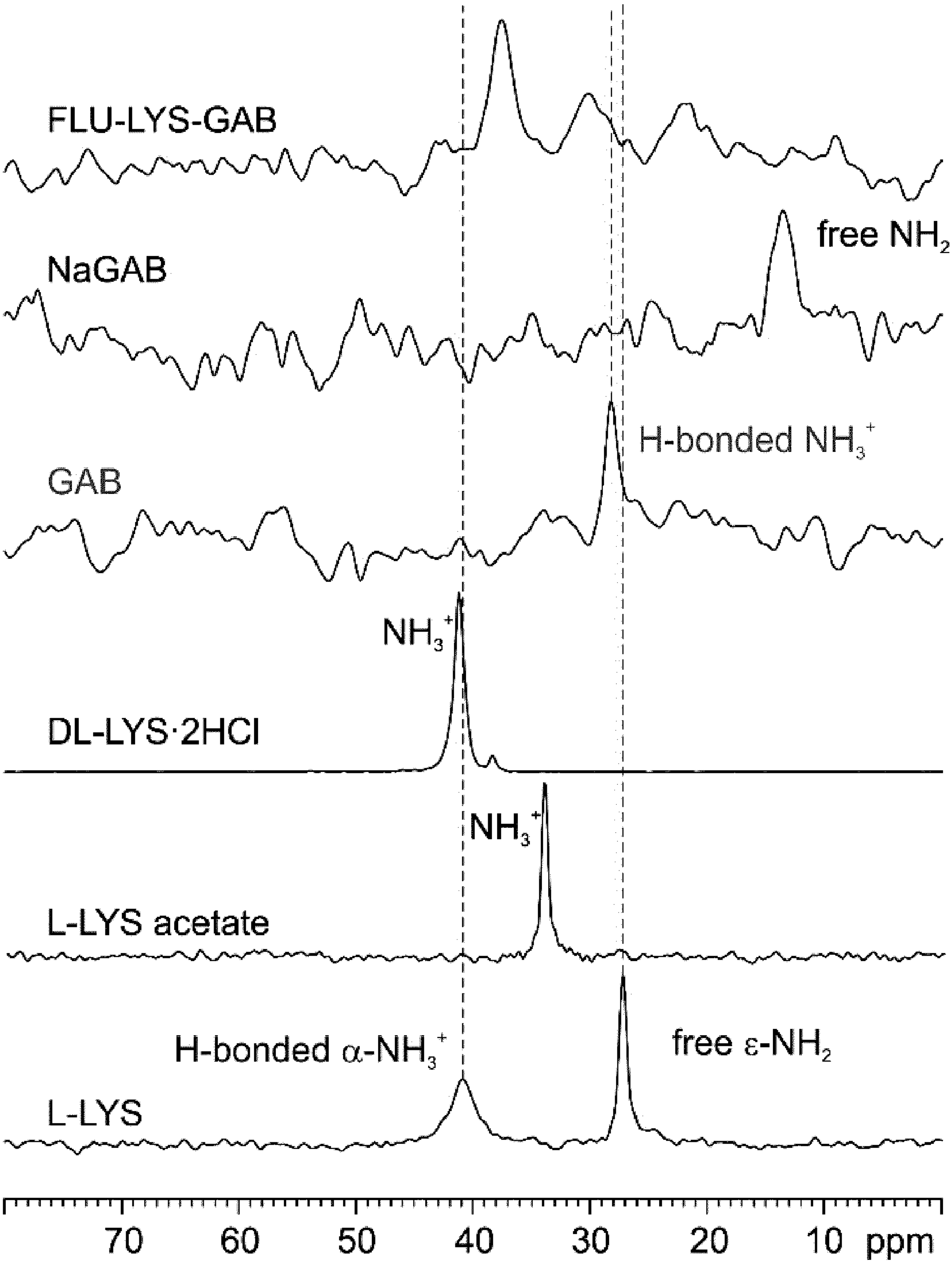
FLU-LYS-GAB
NaGAB
free $NH_2$
GAB
H-bonded $NH_3^+$
$NH_3^+$
DL-LYS·2HCl
$NH_3^+$
L-LYS acetate
H-bonded α-$NH_3^+$
free ε-$NH_2$
L-LYS
70
60
50
40
30
20
10
ppm

FIG.15

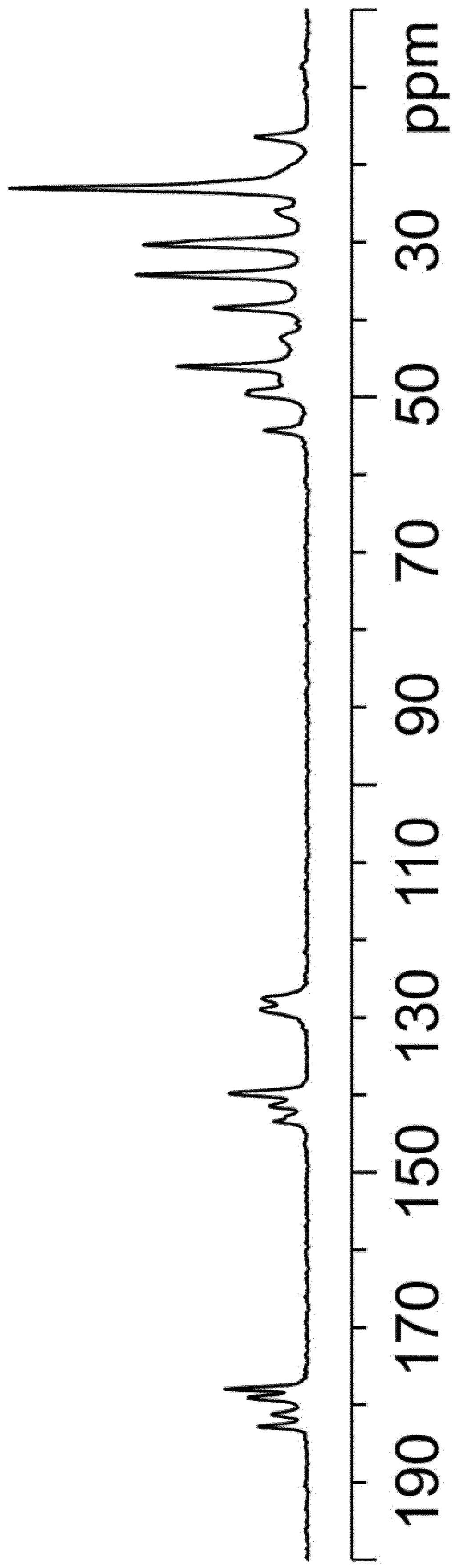


FIG.16

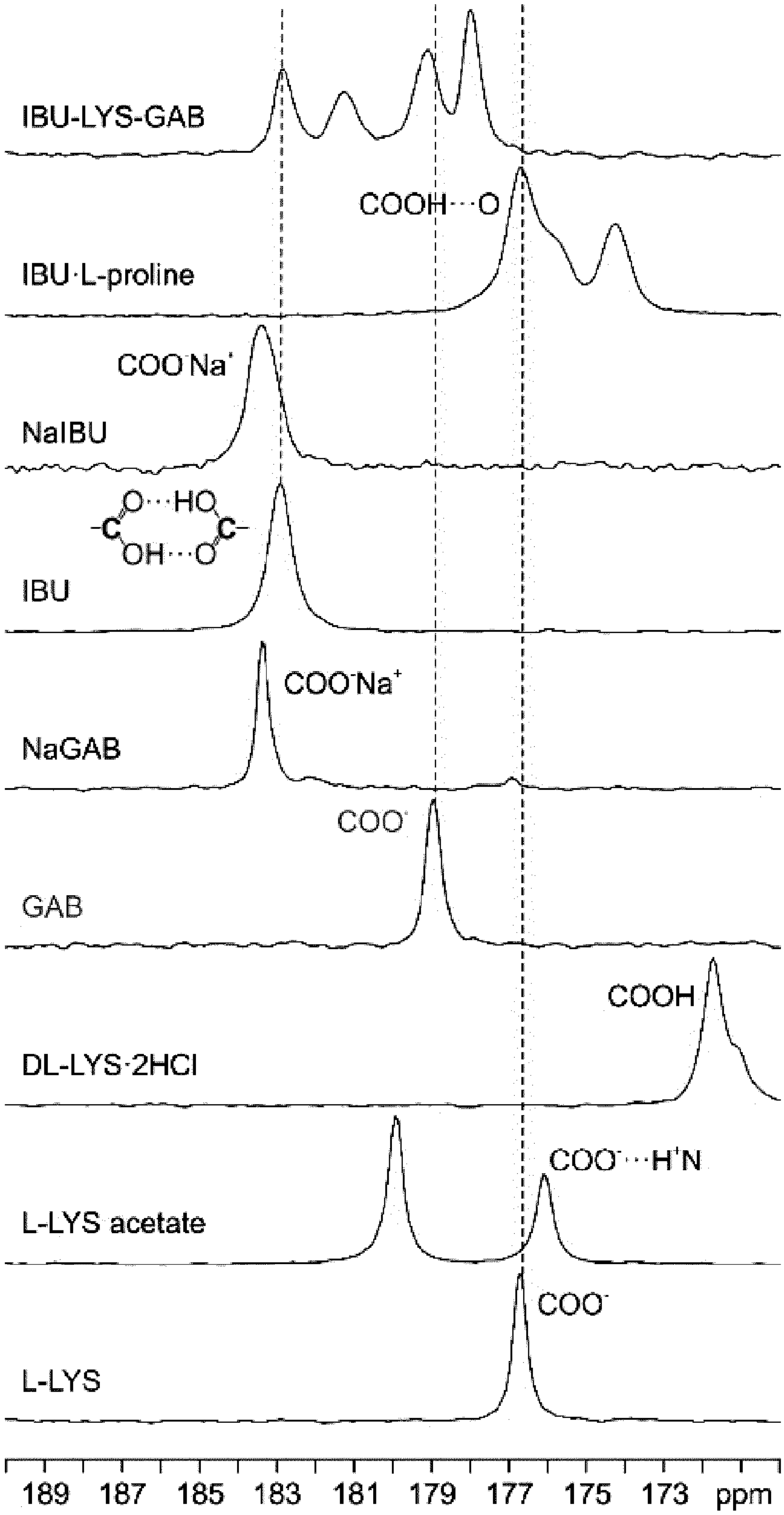
IBU-LYS-GAB
COOH···O
IBU·L-proline
COO⁻Na⁺
NaIBU
IBU
COO⁻Na⁺
NaGAB
COO⁻
GAB
COOH
DL-LYS·2HCl
COO⁻···H⁺N
L-LYS acetate
COO⁻
L-LYS
189 187 185 183 181 179 177 175 173 ppm

FIG.17

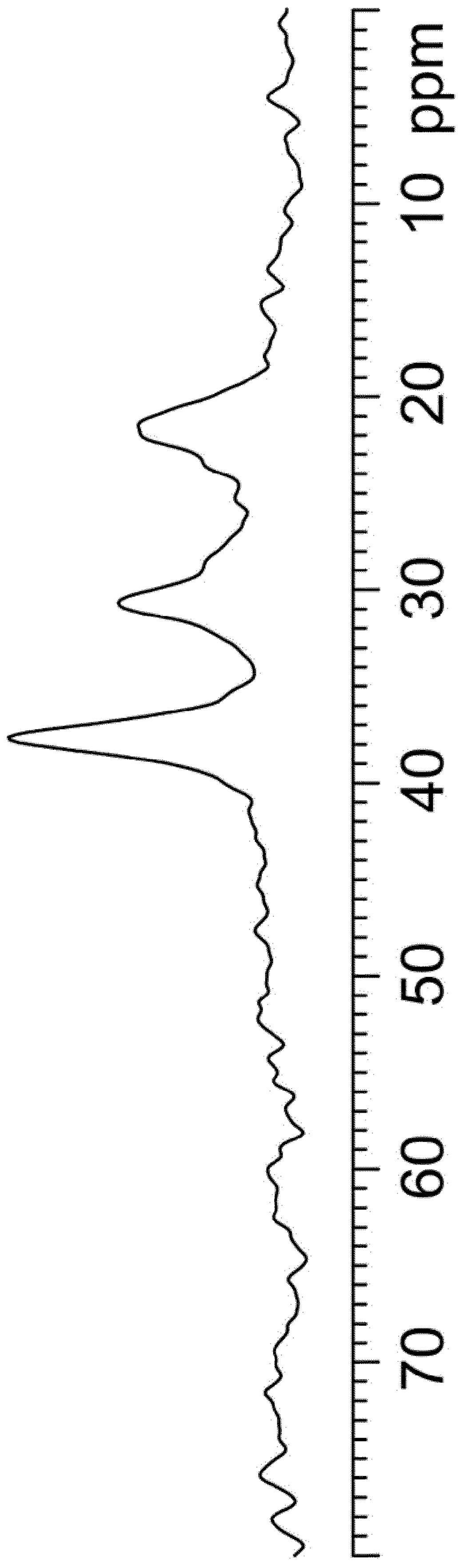


FIG.18

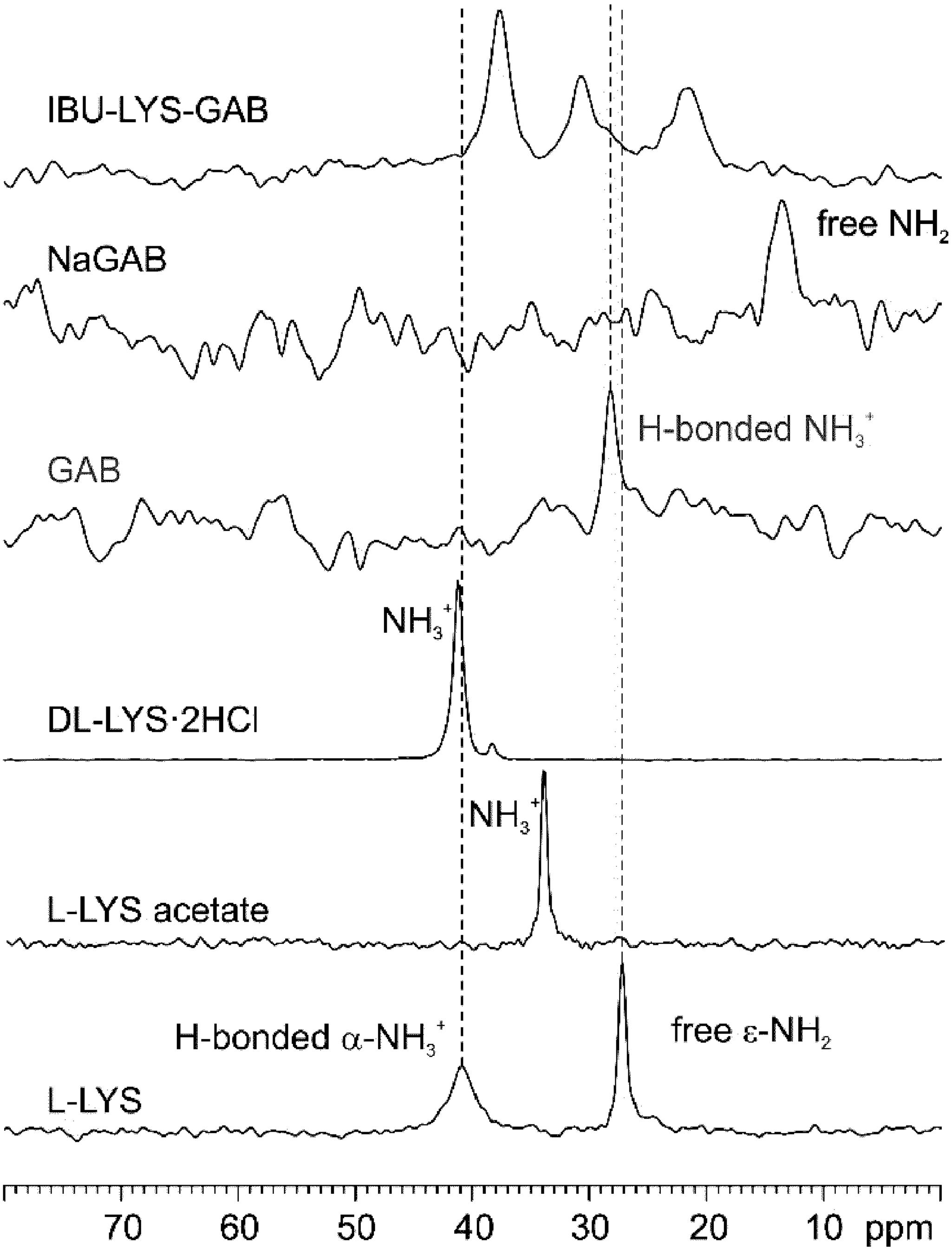
IBU-LYS-GAB
NaGAB
free $NH_2$
GAB
H-bonded $NH_3^+$
DL-LYS·2HCl
$NH_3^+$
L-LYS acetate
$NH_3^+$
H-bonded $\alpha$-$NH_3^+$
free $\varepsilon$-$NH_2$
L-LYS
70
60
50
40
30
20
10
ppm

FIG.19

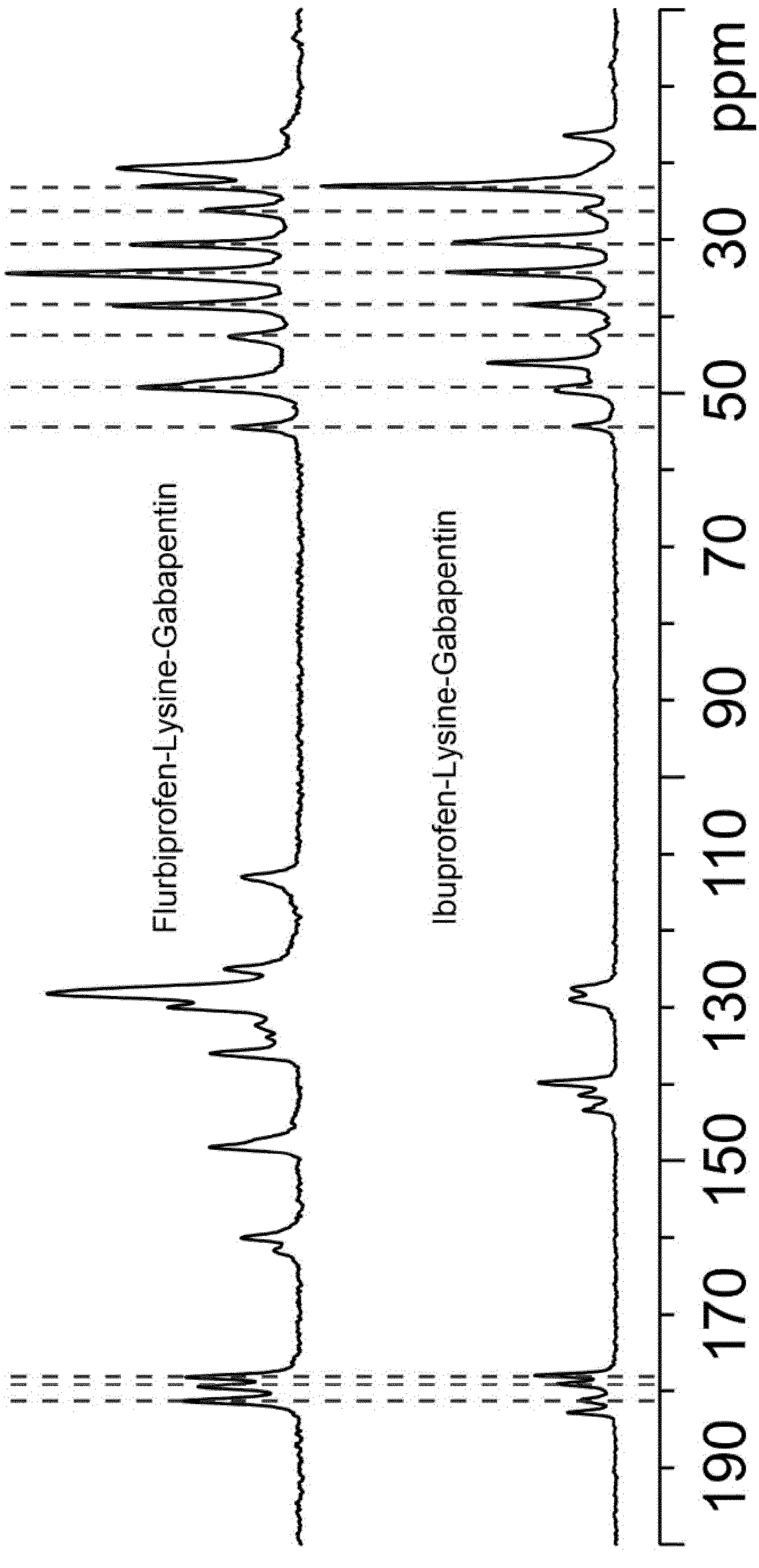
Flurbiprofen-Lysine-Gabapentin
Ibuprofen-Lysine-Gabapentin
ppm
30
50
70
90
110
130
150
170
190

FIG.20

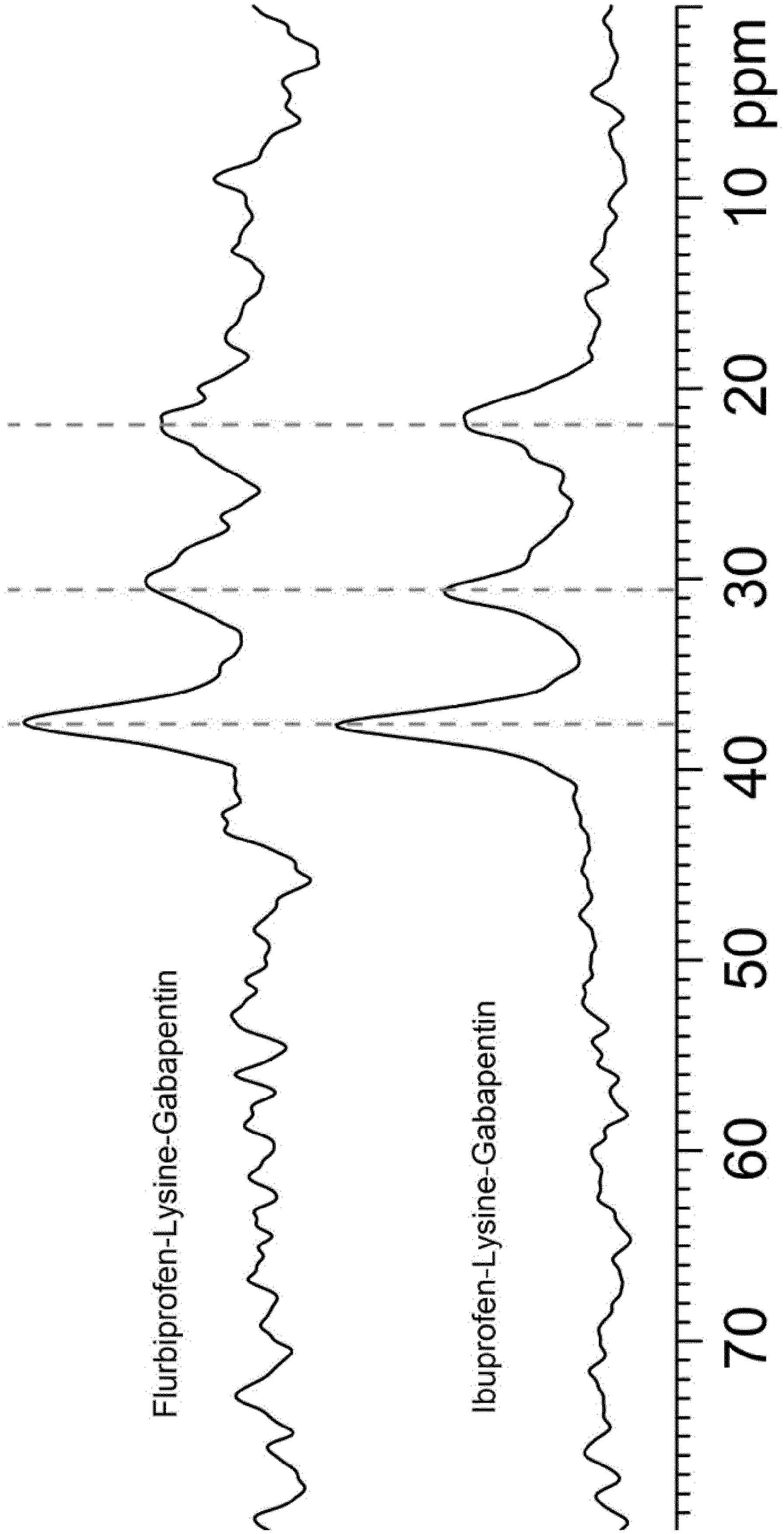
Flurbiprofen-Lysine-Gabapentin
Ibuprofen-Lysine-Gabapentin
ppm
10
20
30
40
50
60
70

FIG.21

FIG.22

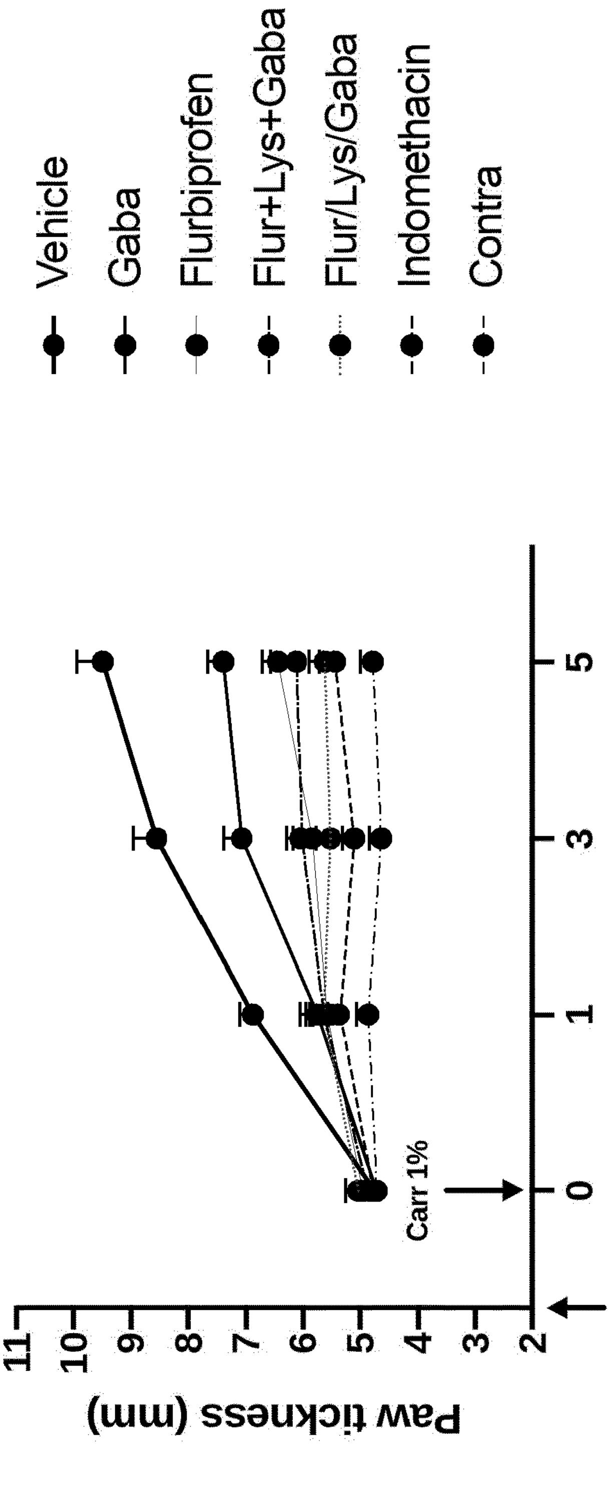
Vehicle
Gaba
Flurbiprofen
Flur+Lys+Gaba
Flur/Lys/Gaba
Indomethacin
Contra
Paw tickness (mm)
11
10
9
8
7
6
5
4
3
2
Carr 1%
0
1
3
5

FIG.23

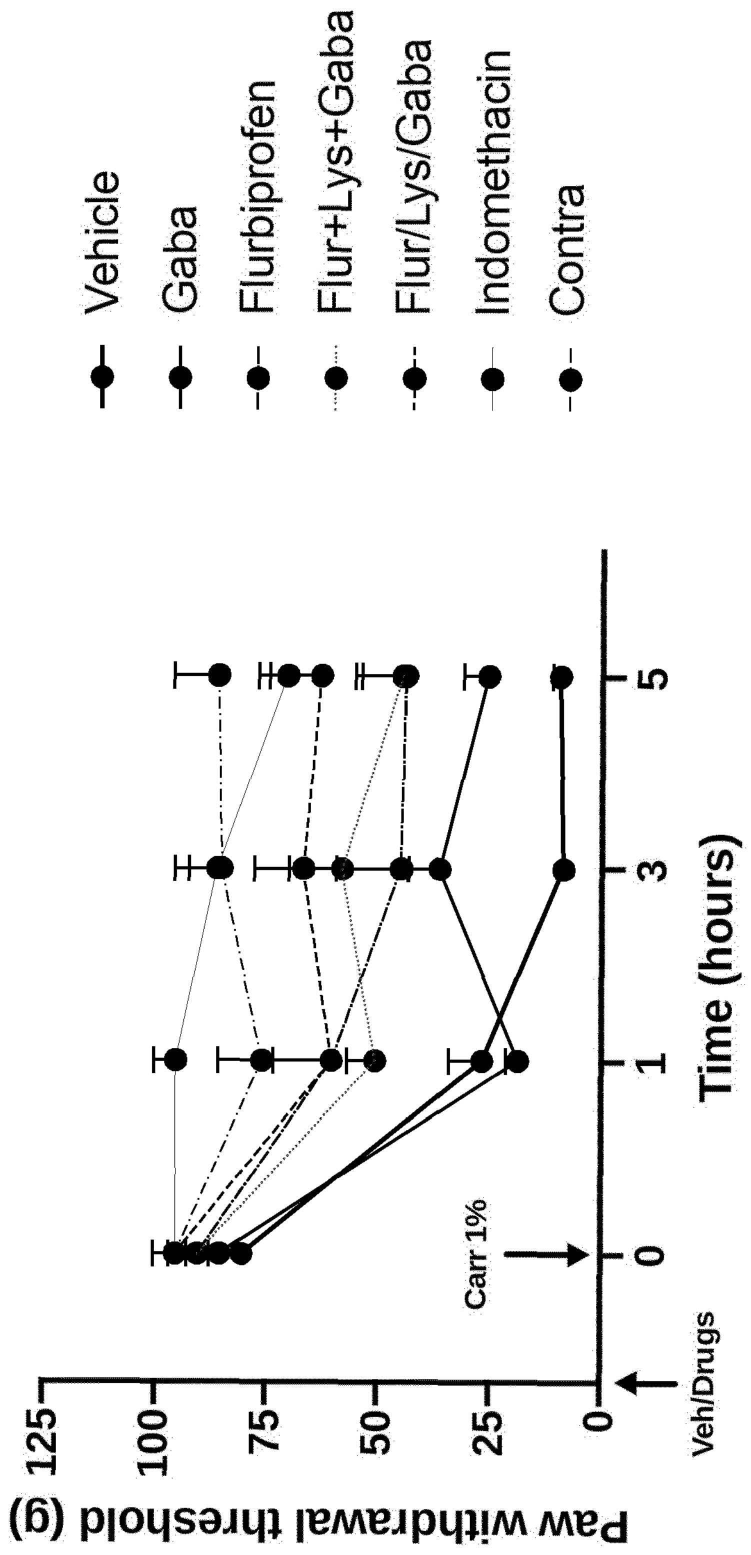
Vehicle
Gaba
Flurbiprofen
Flur+Lys+Gaba
Flur/Lys/Gaba
Indomethacin
Contra
Paw withdrawal threshold (g)
125
100
75
50
25
0
Veh/Drugs
Carr 1%
0
1
3
5
Time (hours)

Europäisches Patentamt
European Patent Office
Office européen des brevets

# EUROPEAN SEARCH REPORT

Application Number
EP 21 20 4515

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ALMANSA CARMEN ET AL: "Co-crystals as a new approach to multimodal analgesia and the treatment of pain", JOURNAL OF PAIN RESEARCH, vol. Volume 12, 1 September 2019 (2019-09-01), pages 2679-2689, XP055906016, DOI: 10.2147/JPR.S208082 Retrieved from the Internet: URL:https://www.dovepress.com/getfile.php?fileID=52528> * See, e.g. page 2683 * ----- | 1-16 | INV. C07C229/26 C07C57/30 C07C229/28 A61P29/00 |
| A | Samineni Ramu: "Effect of Coformers on Novel Co-Crystals of Gabapentin: An In Vivo Approach", , 1 January 2000 (2000-01-01), pages 639-648, XP055906032, Retrieved from the Internet: URL:aaa [retrieved on 2022-03-28] * abstract * ----- | 1-16 | TECHNICAL FIELDS SEARCHED (IPC) C07C A61P |
| A | EP 2 177 215 A1 (ESTEVE LABOR DR [ES]) 21 April 2010 (2010-04-21) * claims 1-4 * ----- | 1-16 | |
| E | WO 2021/214158 A1 (DOMPE FARM SPA [IT]) 28 October 2021 (2021-10-28) * the whole document * ----- | 1-16 | |

The present search report has been drawn up for all claims

1

| Place of search | Date of completion of the search | Examiner |
|---|---|---|
| The Hague | 4 April 2022 | Menchaca, Roberto |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 21 20 4515

This annex lists the patent family members relating to the patent documents cited in the above-mentioned European search report. The members are as contained in the European Patent Office EDP file on
The European Patent Office is in no way liable for these particulars which are merely given for the purpose of information.

04-04-2022

| Patent document cited in search report | Publication date | Patent family member(s) | Publication date |
|---|---|---|---|
| EP 2177215 A1 | 21-04-2010 | AU 2009304235 A1 | 22-04-2010 |
| | | BR PI0920358 A2 | 11-08-2020 |
| | | CA 2737754 A1 | 22-04-2010 |
| | | CN 102186465 A | 14-09-2011 |
| | | CY 1118374 T1 | 28-06-2017 |
| | | DK 2349238 T3 | 12-12-2016 |
| | | EP 2177215 A1 | 21-04-2010 |
| | | EP 2349238 A1 | 03-08-2011 |
| | | ES 2603962 T3 | 02-03-2017 |
| | | HR P20161526 T1 | 16-12-2016 |
| | | HU E030976 T2 | 28-06-2017 |
| | | IL 211785 A | 31-07-2016 |
| | | JP 5645830 B2 | 24-12-2014 |
| | | JP 2012505847 A | 08-03-2012 |
| | | KR 20110069860 A | 23-06-2011 |
| | | LT 2349238 T | 27-12-2016 |
| | | MX 336318 B | 14-01-2016 |
| | | MY 156285 A | 29-01-2016 |
| | | NZ 591873 A | 21-12-2012 |
| | | PL 2349238 T3 | 30-06-2017 |
| | | PT 2349238 T | 24-11-2016 |
| | | RU 2011119608 A | 27-11-2012 |
| | | SM T201600439 B | 10-01-2017 |
| | | UA 109534 C2 | 10-09-2015 |
| | | US 2011257134 A1 | 20-10-2011 |
| | | US 2012172398 A1 | 05-07-2012 |
| | | US 2014057879 A1 | 27-02-2014 |
| | | US 2014350110 A1 | 27-11-2014 |
| | | US 2015196503 A1 | 16-07-2015 |
| | | US 2015196504 A1 | 16-07-2015 |
| | | US 2017000806 A1 | 05-01-2017 |
| | | US 2017027965 A1 | 02-02-2017 |
| | | US 2019231800 A1 | 01-08-2019 |
| | | US 2020171055 A1 | 04-06-2020 |
| | | WO 2010043412 A1 | 22-04-2010 |
| | | ZA 201102762 B | 28-12-2011 |
| WO 2021214158 A1 | 28-10-2021 | EP 3900716 A1 | 27-10-2021 |
| | | WO 2021214158 A1 | 28-10-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2021060421 W


### Non-patent literature cited in the description


- *J Clin Pharmacol,* June 1988, vol. 28 (6), 512-7
- **QUINTERO.** *Journal of Experimental Pharmacology,* 2017, vol. 9, 13-21
- **G. PORTALONE.** *Crystals,* 2020, vol. 10, 999-1012
- **F. Y. WANG ; Q. ZHANG ; Z. ZHANG ; X. GONG ; J. R. WANG ; X. MEI.** *CrystEngComm,* 2018, vol. 20, 5945-5948
- Handbook of Pharmaceutical Excipients. A.H. Kibbe, American Pharmaceutical Association, Washington, USA, and Pharmaceutical Press
- **M. K. MISHRA ; P. SANPHUI ; U. RAMAMURTY ; G. R. DESIRAJU.** *Cryst. Growth Des.,* 2014, vol. 14, 3054-3061
- **P. SANPHUI ; N. R. GOUD ; U. B. R. KHANDAVILLI ; A. NANGIA.** *Cryst. Growth Des.,* 2011, vol. 11, 4135-4145
- **HAJHASHEMI V et al.** The role of central mechanisms in the anti-inflammatory effect of amitriptyline on carrageenan-induced paw edema in rats. *Clinics (Sao Paulo),* 2010
- **CHAPLAN, S. R. et al.** Quantitative assessment of tactile allodynia in the rat paw. *J. Neurosci. Methods,* 1994, vol. 53, 55-63
- **NOUR ELHOUDA SAIDI et al.** Unilateral 6-Hydroxydopamine-Lesioned Rat as Relevant Model to Study the Pain Related to Parkinson's Disease. *NEUROLOGY AND NEUROBIOLOGY,* 2019, ISBN 2613-7828